(19) Europäisches Patentamt
European Patent Office
Office européen des brevets

(11) **EP 3 155 402 B1**

## (12) FASCICULE DE BREVET EUROPEEN

(45) Date de publication et mention de la délivrance du brevet:
**06.11.2019  Bulletin 2019/45**

(21) Numéro de dépôt: **15726641.2**

(22) Date de dépôt: **09.06.2015**

(51) Int Cl.:
*G01N 21/35* (2014.01)          *G01N 33/28* (2006.01)
*G01N 21/359* (2014.01)

(86) Numéro de dépôt international:
**PCT/EP2015/062856**

(87) Numéro de publication internationale:
**WO 2015/189220 (17.12.2015 Gazette 2015/50)**

(54) **PROCÉDÉ DE PRÉPARATION D'UN PRODUIT CIBLE CERTIFIÉ D'UN MÉLANGE DE CONSTITUANTS PAR ANALYSE SPECTRALE**

VERFAHREN ZUR VORBEREITUNG EINER ZERTIFIZIERTEN MISCHUNG AUS BESTANDTEILEN DURCH SPEKTRALANALYSE

METHOD FOR PREPARING A CERTIFIED PRODUCT FROM A MIXTURE OF COMPONENTS BY SPECTRAL ANALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **13.06.2014  EP 14290172**

(43) Date de publication de la demande:
**19.04.2017  Bulletin 2017/16**

(73) Titulaire: **Topnir Systems SAS**
**13100 Aix en Provence (FR)**

(72) Inventeurs:
- **LAMBERT, Didier**
  **F-33430 Bernos Beaulac (FR)**
- **SAINT MARTIN, Claude**
  **F-13330 Pelissane (FR)**
- **SANCHEZ, Miguel**
  **F-13117 Lavera (FR)**
- **RIBERO, Bernard**
  **F-13660 Peyrolles en Provence (FR)**
- **VALLEUR, Marc**
  **F-78152 Le Chesnay Cedex (FR)**

(74) Mandataire: **King, Alex et al**
**Mathisen & Macara LLP**
**Communications House**
**South Street**
**Staines-upon-Thames, Middx TW18 4PR (GB)**

(56) Documents cités:
**EP-A1- 0 801 299          US-A1- 2009 158 824**
**US-A1- 2012 279 114          US-A1- 2012 290 223**

- **ESPINOSA A ET AL: "USE NIR TECHNOLOGY TO OPTIMIZE PLANT OPERATIONS", HYDROCARBON PROCESSING, GULF PUBLISHING CO. HOUSTON, US, 1 février 1995 (1995-02-01), pages 86-89,91/92, XP000615360, ISSN: 0018-8190**
- **MURADOV V G ET AL: "Study of absorption spectra of gasolines and other hydrocarbon mixtures in the second overtone region of the CH3, CH2, CH groups", JOURNAL OF APPLIED SPECTROSCOPY, KLUWER ACADEMIC PUBLISHERS-PLENUM PUBLISHERS, NE, vol. 74, no. 2, 1 mars 2007 (2007-03-01), pages 174-179, XP019528256, ISSN: 1573-8647, DOI: 10.1007/S10812-007-0027-6**

**EP 3 155 402 B1**

**Description**

La présente invention concerne un procédé de certification et d'optimisation d'un mélange de constituants pour l'obtention d'un produit cible par analyse spectrale, de préférence par analyse spectrale (topologique) dans le proche infrarouge (« NIR »).

En particulier, la présente invention concerne la régulation des mélanges de constituants par batch ou en ligne, tels que par exemple des mélanges de produits pétroliers ou autres, dans lesquels les dits mélanges sont conformes à un ensemble de spécifications significatives.

Plus particulièrement, la présente invention concerne un procédé de certification et d'optimisation d'un mélange de constituants pour l'obtention d'un produit cible par analyse spectrale, par exemple par RMN, Raman, IR, et/ou UV/Visible, de préférence par analyse spectrale (topologique) dans le proche infrarouge (« NIR »), sous contrainte, la dite contrainte étant basée

- sur l'utilisation privilégiée d'au moins un des constituants du mélange, et/ou
- sur la modification d'au moins une caractéristique du produit cible.

Lorsqu'on effectue un mélange de constituants divers, l'objectif est d'obtenir un produit cible présentant une gamme de valeurs de caractéristiques physico-chimiques définies à l'avance et permettant d'en garantir la certification. A titre d'exemple, pour un mélange de produits pétroliers, par exemple un carburant, ces caractéristiques pourront être son indice d'octane, son indice de cétane, la tenue au froid, la teneur en composés polyaromatiques, la tension de vapeur, etc...

L'article, "USE NIR TECHNOLOGY TO OPTIMIZE PLANT OPERATIONS"; HYDROCARBON PROCESSING; 1995-02-01; ESPINOSA A ET AL; pages 86-92" divulgue un procédé de préparation d'un mélange d'un produit hydrocarbure tel qu'un mélange brut ou d'essence, dans lequel des capteurs NIR sont utilisés pour détecter le spectre du mélange du produit ainsi que les spectres des constituants initiaux utilisés pour produire le mélange par un mélangeur. La méthode utilise le contrôle de mélange multivarié au moyen d'un système de retour selon lequel des signaux d'un analyseur en ligne ainsi que des données concernant les constituants mesurées avec un analyseur hors ligne sont alimentés a un système de contrôle distribué (DCS) qui communique avec une unité de logiciel de contrôle multivarié qui effectue des calculs du mélange.

La présente invention s'appliquera en particulier au mélange de constituants de produits pétroliers cibles (par exemple des carburants) dans tout lieu approprié, par exemple une raffinerie, un dépôt pétrolier et/ou tout dispositif utilisant un produit pétrolier constitué d'un mélange de constituants préparé par batch et/ou en ligne.

Ainsi, dans un dépôt pétrolier et/ou une raffinerie de pétrole, des carburants pour moteur peuvent être réalisés par la technique dite du mélange par batch et/ou en ligne, selon laquelle les différents constituants liquides, ainsi que les éventuels additifs, sont introduits dans un bac et/ou une conduite servant de mélangeur. Cette introduction des différents constituants peut être effectuée de manière simultanée ou non. Ce mélange des différents constituants peut être effectué par batch et/ou en continu. Le débit des différents constituants et/ou la concentration des différents constituants sont généralement commandés et contrôlés par un ordinateur et la durée de préparation d'un lot de carburant peut prendre plusieurs dizaines d'heures.

C'est à ce type d'installation industrielle que l'on se référera plus précisément dans la suite de la présente description, sans que ceci n'implique une limitation du champ d'application de l'invention. Les propriétés du produit cible (par exemple un carburant) fabriqué sont contrôlées à diverses reprises pendant la fabrication et des analyses sont effectuées dans ce but sur des échantillons prélevés en sortie du mélangeur et/ou dans l'enceinte de stockage en cours de remplissage. A partir des résultats de ces analyses, les débits et/ou les concentrations des constituants du mélange ainsi que des éventuels additifs sont ajustés pour aligner les valeurs mesurées avec les valeurs de consigne. Pour chaque analyse, il y a avantage à limiter autant que possible l'intervalle de temps séparant le prélèvement d'un échantillon du mélange en cours de préparation et l'obtention de la valeur mesurée ; bien que les analyseurs « en ligne » puissent en théorie répondre à ce besoin, on a constaté que l'évolution rapide des constituants et des caractéristiques des produits cibles ne permettaient pas toujours d'être pleinement efficace et qu'il fallait encore bien souvent échantillonner et procéder à des analyses dites « off-line ».

En effet, une particularité des constituants mélangés pour la préparation du produits cible est qu'ils peuvent soit provenir de sources différentes d'approvisionnement et/ou tout simplement varier en terme de qualité et/ou de caractéristiques physico-chimiques au fil du temps. Cette particularité est apparue d'autant plus critique ces dernières années de par les effets de la mondialisation et de la multiplicité d'accès à de nouvelles sources d'approvisionnement. Un besoin se fait donc sentir dans la technique de disposer d'un moyen amélioré de préparation de produits pétroliers qui réponde plus efficacement à ces nouvelles exigences.

Une autre particularité des produits pétroliers cibles est également apparue ces dernières années. Les évolutions environnementales ont conduit à l'adoption de nouvelles normes et/ou à des changements permanents de certaines

2

caractéristiques des produits pétroliers cibles. On citera à titre d'exemple, la production temporaire de carburants moins polluants lors de pics de pollution et/ou la teneur en composants bio des dits carburants. Un besoin se fait donc sentir dans la technique de disposer d'un moyen amélioré de préparation et de certification de produits pétroliers qui réponde plus efficacement à ces nouvelles exigences.

Ce sont ces problèmes que se propose de résoudre la présente invention par l'utilisation d'un procédé et/ou d'un dispositif de certification et d'optimisation d'un mélange de constituants pour l'obtention d'un produit cible par analyse spectrale, par exemple par RMN, Raman, IR, NIR et/ou UV/Visible, de préférence par analyse spectrale (topologique) dans le proche infrarouge (« NIR »).

En particulier, la présente invention concerne la régulation des mélanges de constituants par batch et/ou en ligne, tels que par exemple des mélanges de produits pétroliers, dans lesquels les dits mélanges sont conformes à un ensemble de spécifications significatives.

Plus particulièrement, la présente invention concerne un procédé et un dispositif de certification et d'optimisation d'un mélange de constituants pour l'obtention d'un produit cible par analyse spectrale, par exemple par RMN, Raman, IR, NIR et/ou UV/Visible, de préférence par analyse spectrale (topologique) dans le proche infrarouge (« NIR ») sous contrainte, la dite contrainte étant basée

- sur l'utilisation privilégiée d'au moins un des constituants du mélange, et/ou
- sur la modification d'au moins une caractéristique du produit cible et/ou
- l'amélioration d'au moins une propriété du produit cible, la dite propriété faisant partie des conditions de certification du dit produit cible.

PROCEDE M

Ainsi, la présente invention concerne un procédé M de certification et de préparation d'un produit cible, par exemple un produit pétrolier cible, par exemple un carburant, selon le procédé de la revendication 1.

Selon un mode d'exécution de la présente invention, la valeur de la dite donnée spectrale du produit cible ainsi préparé est obtenue par calcul lors de l'étape 3 lors de la reconstitution de la donnée spectrale de mélange.

Selon un mode d'exécution de la présente invention, la valeur de la dite donnée spectrale du produit cible ainsi préparé est obtenue par mesure spectrale du produit ainsi obtenu.

Selon un mode d'exécution de la présente invention, la valeur de la dite donnée spectrale du produit cible ainsi préparé est obtenue à la fois par calcul lors de l'étape 3 lors de la reconstitution de la donnée spectrale de mélange, et également par mesure spectrale du produit ainsi obtenu.

La présente invention est donc caractérisée par le fait qu'on dispose d'au moins une donnée spectrale caractérisant le produit cible et qui définit son domaine spectral, la valeur de la dite donnée spectrale répondant à au moins une condition du procédé de certification. Ces données spectrales sont de préférence des données mesurées au moyen du même type d'analyse spectrale, de préférence au moyen du même type de spectromètre ; ces données spectrales peuvent par exemple être des « spectres ».

Le produit cible selon la présente invention doit donc posséder en vue de sa commercialisation d'un ensemble de gammes de valeurs de caractéristiques physico-chimiques qui sont dictées par le procédé de certification ; ces gammes de valeurs de caractéristiques physico-chimiques sont en général définies au moyen de gammes de « propriétés » du produit cible, des exemples des dites propriétés étant définis plus en détail dans la suite de la présente description. Ainsi, dans un mode d'exécution de la présente invention, une condition du procédé de certification peut avantageusement signifier une gamme de valeurs d'une propriété « X » du produit cible, la dite propriété « X » faisant partie des propriétés requises par le procédé de certification du produit cible; ainsi, dans ce mode d'exécution, à la valeur de la donnée spectrale de l'étape 1 correspond une valeur de la propriété « X » qui elle-même répond à une condition du procédé de certification. La présente invention permet donc au moyen de données spectrales non seulement de contrôler les concentrations et/ou débits des constituants alimentant le mélangeur de manière à préparer le produit cible mais également de s'assurer que le produit cible répond aux conditions/contraintes du processus de certification.

Selon un mode d'exécution de la présente invention, le spectre du produit cible ainsi préparé est obtenu par calcul lors de l'étape 3 lors de la reconstitution du spectre de mélange.

Selon un mode d'exécution de la présente invention, le spectre du produit cible ainsi préparé est obtenu par mesure spectrale du produit ainsi obtenu.

Selon un mode d'exécution de la présente invention, le spectre du produit cible ainsi préparé est obtenu à la fois par calcul lors de l'étape 3 lors de la reconstitution du spectre de mélange, et également par mesure spectrale du produit ainsi obtenu.

Le domaine spectral caractérisant le produit cible peut être déterminé par toute méthode appropriée. A titre d'exemple, ce domaine sera déterminé aux moyen des agrégats tels que décrits ci-après dans la description. La caractéristique fondamentale du domaine spectral est qu'il définit le fait que le mélange final soit conforme à un ensemble de spécifications significatives du produit cible ; c'est ce qui permet de dire que le mélange est à la spec (« on-spec » en langue anglaise) selon le jargon utilisé par l'homme du métier.

Le produit cible (par exemple un produit pétrolier) contient généralement un constituant majeur dont la concentration en poids dans le produit cible est la plus élevée. Selon un mode d'exécution particulier et préféré de la présente invention, au moins un des spectres caractérisant un des constituants mineurs (c'est-à-dire tout constituant non majeur) du produit cible de l'étape 2 susmentionnée est un spectre obtenu par analyse d'un mélange du dit constituant mineur avec soit le dit constituant majeur, soit un mélange représentatif du produit cible.

Selon un mode d'exécution de la présente invention, le nombre de constituants « n » du produit cible est supérieur ou égal à 2, par exemple supérieur ou égal à 3.

Comme déjà indiqué, les données spectrales sont de préférence des données mesurées au moyen du même type d'analyse spectrale, de préférence au moyen du même type de spectromètre ; ces données spectrales peuvent par exemple être tout type approprié de grandeurs spectrales constitutives d'une banque de données spectrales correspondante. Ces grandeurs spectrales peuvent être tous types de signaux caractérisant les spectres, par exemple les absorbances, transmittances, réflectances, etc... ; les absorbances ou densités optiques étant les signaux les plus communément utilisés. A titre d'exemple, on citera également comme signaux les dérivées des absorbances ou encore tout autre mesure résultant d'un autre type de traitement mathématique des dites absorbances.

La présente invention est donc caractérisée par le fait qu'on dispose d'au moins un spectre caractérisant le produit cible et qui définit son domaine spectral, le dit spectre répondant à au moins une condition du procédé de certification. Ces spectres sont de préférence des données mesurées au moyen du même type d'analyse spectrale, de préférence au moyen du même type de spectromètre.

Le produit cible selon la présente invention doit donc posséder en vue de sa commercialisation d'un ensemble de gammes de valeurs de caractéristiques physico-chimiques qui sont dictées par le procédé de certification ; ces gammes de valeurs de caractéristiques physico-chimiques sont en général définies au moyen de gammes de « propriétés » du produit cible, des exemples des dites propriétés étant définis plus en détail dans la suite de la présente description. Ainsi, dans un mode d'exécution de la présente invention, une condition du procédé de certification peut avantageusement signifier une gamme de valeurs d'une propriété « X » du produit cible, la dite propriété « X » faisant partie des propriétés requises par le procédé de certification du produit cible; ainsi, dans ce mode d'exécution, au spectre de l'étape 1 correspond une valeur de la propriété « X » qui elle-même répond à une condition du procédé de certification. La présente invention permet donc au moyen du spectre non seulement de contrôler les concentrations et/ou débits des constituants alimentant le mélangeur de manière à préparer le produit cible mais également de s'assurer que le produit cible répond aux conditions/contraintes du processus de certification.

Selon un mode d'exécution de la présente invention les données spectrales constituent une (ou plusieurs) banque(s) de données spectrales.

PROCEDE M2 - Banque de données spectrales

Ainsi, selon un mode d'exécution particulier, la présente invention concerne également un procédé M2 de certification et de préparation d'un produit cible, par exemple un produit pétrolier cible, par exemple un carburant, par mélange par batch et/ou en ligne de ses « n » constituants à partir de différents flux des dits constituants à des concentrations et/ou des débits contrôlés, avec incorporation optionnelle d'additifs, ledit produit cible devant posséder en vue de sa commercialisation un ensemble de gammes de valeurs de caractéristiques physico-chimiques, procédé dans lequel on alimente un mélangeur par batch et/ou en continu avec les dits constituants à des concentrations et/ou des débits contrôlés, cette méthode étant caractérisée en ce que:

    1. on dispose d'une banque de données spectrales caractérisant le produit cible et qui définit son domaine spectral, la dite banque de données spectrales répondant à au moins une condition du procédé de certification,

    2. on dispose de banques de données spectrales caractérisant chacune de manière individuelles au moins deux - de préférence tous les « n » - constituants du produit cible,

    3. on utilise un programme informatique permettant de calculer les gammes de proportions respectives des dits constituants nécessaires à la reconstitution, à partir des banques de données spectrales de l'étape 2, d'une banque de données spectrales de mélange appartenant au domaine spectral de l'étape 1,

    4. on utilise les gammes de proportions respectives des constituants de l'étape 3 pour contrôler les concentrations et/ou débits des constituants alimentant le mélangeur de manière à préparer le produit cible dont la banque de données spectrales répond à la même condition (ou aux mêmes conditions) du procédé de certification de la première étape.

Selon un mode d'exécution de la présente invention, la banque de données spectrales du produit cible ainsi préparé est obtenue par calcul lors de l'étape 3 lors de la reconstitution de la banque de données spectrales de mélange.

Selon un mode d'exécution de la présente invention, la banque de données spectrales du produit cible ainsi préparé est obtenue par mesure spectrale du produit cible ainsi obtenu.

Selon un mode d'exécution de la présente invention, la banque de données spectrales du produit cible ainsi préparé est obtenue à la fois par calcul lors de l'étape 3 lors de la reconstitution de la banque de données spectrales de mélange, et également par mesure spectrale du produit cible ainsi obtenu.

Le domaine spectral caractérisant le produit cible peut être déterminé par toute méthode appropriée. A titre d'exemple, ce domaine sera déterminé aux moyen des agrégats tels que décrits ci-après dans la description. La caractéristique fondamentale du domaine spectral est qu'il définit le fait que le mélange final soit conforme à un ensemble de spécifications significatives du produit cible ; c'est ce qui permet de dire que le mélange est à la spec (« on-spec » en langue anglaise) selon le jargon utilisé par l'homme du métier.

Selon un mode d'exécution particulier de la présente invention, après la deuxième étape, on constitue, à partir des banques de données spectrales des constituants, des banques de données spectrales 2BIS qui caractérisent les mélanges des dits constituants, et qui sont utilisées pour l'étape 3.

PROCEDE M2BIS

Ainsi, selon un mode d'exécution particulier, la présente invention concerne également un procédé M2BIS de certification et de préparation d'un produit cible, par exemple un produit pétrolier cible, par exemple un carburant, par mélange par batch et/ou en ligne de ses « n » constituants à partir de différents flux des dits constituants à des concentrations et/ou des débits contrôlés, avec incorporation optionnelle d'additifs, ledit produit cible devant posséder en vue de sa commercialisation un ensemble de gammes de valeurs de caractéristiques physico-chimiques, procédé dans lequel on alimente un mélangeur par batch et/ou en continu avec les dits constituants à des concentrations et/ou des débits contrôlés, ce procédé étant caractérisée en ce que:

1. on dispose d'une banque de données spectrales caractérisant le produit cible et qui définit son domaine spectral, la dite banque de données spectrales répondant à au moins une condition du procédé de certification,
2. on dispose de banques de données spectrales caractérisant chacun de manière individuelles au moins deux - de préférence tous les « n » - constituants du produit cible,
2BIS. on constitue, à partir des banques de données spectrales des constituants de l'étape 2, des banques de données spectrales (2bis) qui caractérisent les mélanges des dits constituants,
3. on utilise un programme informatique permettant d'identifier dans les banques de données spectrales de l'étape 2BIS la(les) banque(s) de données spectrales de mélange appartenant au domaine spectral de l'étape 1 et permettant ainsi de définir les gammes de proportions respectives des constituants,
4. on utilise les gammes de proportions respectives des constituants de l'étape 3 pour contrôler les concentrations et/ou débits des constituants alimentant le mélangeur de manière à préparer le produit cible dont la banque de données spectrales répond à la même condition (ou aux mêmes conditions) du procédé de certification de la première étape.

Comme déjà décrit ci-avant, le domaine spectral caractérisant le produit cible peut être déterminé par toute méthode appropriée. A titre d'exemple, ce domaine sera déterminé aux moyen des agrégats tels que décrits ci-après dans la description. La caractéristique fondamentale du domaine spectral est qu'il définit le fait que le mélange final soit conforme à un ensemble de spécifications significatives du produit cible ; c'est ce qui permet de dire que le mélange est à la spec (« on-spec » en langue anglaise) selon le jargon utilisé par l'homme du métier.

La présente invention est donc caractérisée par le fait qu'on dispose d'au moins une banque de données spectrales caractérisant le produit cible et qui définit son domaine spectral, la dite banque de données spectrales répondant à au moins une condition du procédé de certification. Cette banque de données spectrales provient de préférence de mesures effectuées au moyen du même type d'analyse spectrale, de préférence au moyen du même type de spectromètre.

Le produit cible doit donc posséder en vue de sa commercialisation d'un ensemble de gammes de valeurs de caractéristiques physico-chimiques qui sont dictées par le procédé de certification ; ces gammes de valeurs de caractéristiques physico-chimiques sont en général définies au moyen de gammes de « propriétés » du produit cible, des exemples des dites propriétés étant définis plus en détail dans la suite de la présente description. Ainsi, dans un mode d'exécution de la présente invention, une condition du procédé de certification peut avantageusement signifier une gamme de valeurs d'une propriété « X » du produit cible, la dite propriété « X » faisant partie des propriétés requises par le procédé de certification du produit cible; ainsi, dans ce mode d'exécution, à la banque de données spectrales de l'étape 1 correspond une valeur de la propriété « X » qui elle-même répond à une condition du procédé de certification. La présente invention permet donc au moyen de banques de données spectrales non seulement de contrôler les concentrations et/ou débits des constituants alimentant le mélangeur de manière à préparer le produit cible mais également de s'assurer que le produit cible répond aux conditions/contraintes du processus de certification.

Comme déjà décrit ci-avant, le produit cible (par exemple un produit pétrolier) contient généralement un constituant majeur dont la concentration en poids dans le produit cible est la plus élevée. Selon un mode d'exécution particulier et préféré de la présente invention, au moins une des banques de données spectrales caractérisant un des constituants mineurs du produit cible de l'étape 2 susmentionnée est une banque de données spectrales obtenue par analyse d'un mélange du dit constituant mineur (c'est-à-dire tout constituant non majeur) avec soit le dit constituant majeur, soit un mélange représentatif du produit cible.

Selon un mode d'exécution préféré de la présente invention, la méthode d'analyse spectrale utilisée est choisie parmi, par exemple la RMN, le Raman, l'IR, le NIR et/ou l'UV/Visible, de préférence par analyse spectrale topologique dans le proche infrarouge (« NIR »).

Selon un mode d'exécution préféré de la présente invention, le procédé de certification et de préparation d'un produit cible par mélange en ligne de ses constituants permet également de tenir compte d'un ensemble de contraintes nouvelles et ainsi d'optimiser le dit procédé.

La Demanderesse a prouvé que l'utilisation de l'analyse spectrale telle que décrite ci-dessus et, en particulier l'utilisation de l'analyse spectrale dans le proche infrarouge, a permis d'améliorer considérablement la fiabilité et surtout le temps de réponse des procédés de certification et de préparation de produits cibles.

Les nombreux avantages qu'apportent la présente invention seront à présent décrits au moyen d'un cas pratique ; le dit cas pratique est purement illustratif ce qui signifie que ce qui est décrit ci-après ne limite pas la portée de la présente invention, portée qui est définie par les revendications.

Prenons comme produit cible une essence de type SP98 également appelée supercarburant sans plomb 98. En vue de sa commercialisation cette essence doit donc posséder un ensemble de gammes de valeurs de caractéristiques physico-chimiques qui sont dictées par le procédé de certification ; ces gammes de valeurs de caractéristiques physico-chimiques sont en général définies au moyen de gammes de « propriétés » [Px] du produit cible, des exemples des dites propriétés [Px] étant définies ci-dessous (il est donc évident que ces propriétés peuvent évoluer dans le temps, que ce soit en fonction des pays, des saisons (été, hiver), ou également en fonction de l'évolution des matières premières, des carburants, des additifs ou encore des moteurs) :

- P1 = Pression de Vapeur Reid [« Reid Vapor Pressure (RVP) »] avec valeurs maximales à ne pas dépasser (exemple de dimension « kPa » ; exemples de valeur maximale : 60kPa en été, 90kPa en hiver). Cette propriété est une mesure de la volatilité de l'essence. Elle est importante car elle impacte sur le fonctionnement du moteur à essence des véhicules. Par exemple, des niveaux élevés de vaporisation sont souhaitables pour le démarrage et le fonctionnement en hiver alors que des niveaux minimas sont souhaitables pendant les chaleurs estivales. Ainsi, les raffineries de pétrole et/ou terminaux pétroliers et/ou terminaux de carburants manipulent la pression de vapeur Reid saisonnière pour maintenir la fiabilité du moteur à essence ;
- P2 = masse volumique (ou densité) dont la valeur doit être comprise dans une gamme définie par la certification en vigueur (exemple de dimension « Kg/m3 » ; exemples de gamme : 720 - 775) ;
- P3 = propriété de distillation - pourcentage d'évaporé à 70 °C (« E70 ») avec valeurs maximales à ne pas dépasser (exemples de valeur maximale : 48 % Vol. max en été, 50 % Vol. max en hiver) ;
- P4 = Indice d'octane moteur (Motor Octane Number "MON") avec des valeurs minima (exemple Min 87.0) ;
- P5 = Indice d'octane recherche (Research Octane Number "RON") avec des valeurs minima (exemple Min 95.0) ;
- P6 = teneur en Benzène avec valeurs maximales à ne pas dépasser (exemple 1 % Vol. max) ;
- P7 = teneur en Oléfines avec valeurs maximales à ne pas dépasser (exemple 18 % Vol. max) ;
- P8 = teneur en Aromatiques avec valeurs maximales à ne pas dépasser (exemple 35 % Vol. max) ;
- P9 = teneur en Oxygène avec valeurs maximales à ne pas dépasser (exemple 2.7 % Vol. max) ;
- P10 = teneur en Ethers avec valeurs maximales à ne pas dépasser (exemple 22 % Vol. max).

Selon un mode d'exécution préféré de la présente invention, les données spectrales susmentionnées permettent donc simultanément

- de définir les gammes de proportions respectives des constituants nécessaires à la préparation d'un produit cible, et
- d'identifier parmi ces gammes de proportions respectives des constituants les sous-gammes permettant la préparation du produit cible dont la valeur d'au moins une des propriétés Px (qui est de préférence mesurée et/ou calculée au moyen des mêmes données spectrales) satisfait la contrainte du procédé de certification.

Le fait que les données spectrales permettent la préparation et la certification du produit cible a avantageusement ouvert de nombreuses possibilités d'applications additionnelles selon la présente invention.

Selon un mode d'exécution préféré de la présente invention, au moins deux propriétés Px, par exemple au moins trois, quatre, cinq, six, sept, huit, neuf, dix propriétés (par exemple avantageusement sélectionnées parmi les propriétés susmentionnées) sont utilisées pour satisfaire la contrainte du procédé de certification du produit cible ; les dites propriétés ayant été mesurées et/ou calculées en utilisant les données spectrales qui ont servi à définir les gammes de proportions respectives des constituants nécessaires à la préparation d'un produit cible.

Ainsi, dans un mode d'exécution particulier de la présente invention, lorsqu'on désirera privilégier [par exemple minimiser, maximiser et/ou moyenner] une (ou plusieurs) propriété(s) « Px », le procédé de certification et de préparation inclura une étape additionnelle qui consistera à sélectionner parmi les dites gammes de proportions respectives des constituants alimentant le mélangeur, la gamme de proportions à laquelle correspondra la valeur privilégiée de la propriété « Px ». La sélection d'une valeur privilégiée de la propriété « Px » [qu'elle soit minimisée, maximisée et/ou moyennée] peut être dictée pour diverses raisons parmi lesquelles on citera à titre illustratif des raisons économiques et/ou des raisons de saisons (été/hiver) et/ou des raisons liées aux évolutions des produits pétroliers et/ou des raisons de gestion améliorée de la logistique liée aux approvisionnements.

Bien que la présente invention puisse avantageusement se substituer à la majorité des méthodes conventionnelles utilisées actuellement pour le procédé de certification et de préparation d'un produit cible, il peut arriver que certaines propriétés Pz exigées pour la certification du produit cible ne puissent pas être déterminées en utilisant les données spectrales de la présente invention ; ainsi, la présente invention pourra avantageusement être combinée avec une ou plusieurs étapes additionnelles consistant à utiliser une ou plusieurs méthodes conventionnelles de mesure et/ou de vérification de(s) la propriété(s) Pz de manière à sélectionner, parmi les dites gammes de proportions respectives des constituants alimentant le mélangeur, la gamme de proportions à laquelle correspond un produit cible dont la(les) propriété(s) Pz satisfait à la contrainte dictée par la certification du dit produit cible. A titre d'exemple illustratif de propriété Pz, nous citerons la teneur en soufre qui peut être mesurée - de préférence en ligne - au moyen de toute méthode conventionnelle appropriée, par exemple par détermination par spectrométrie de fluorescence X dispersive en longueur d'onde de la teneur en soufre dans les produits pétroliers.

CONTRAINTE « X »

Dans un mode d'exécution particulier de la présente invention, lorsqu'on désirera privilégier l'utilisation du constituant « X » parmi les « n » constituants du mélange produit cible, la méthode de certification et de préparation inclura une étape additionnelle qui consistera à sélectionner parmi les dites gammes de proportions respectives des constituants alimentant le mélangeur, la gamme de proportion ayant la concentration en constituant « X » la plus élevée. La contrainte d'utilisation du constituant « X » peut être privilégiée (ou non) pour diverses raisons parmi lesquelles on citera à titre illustratif des raisons économiques et/ou des raisons de nettoyage périodique du récipient de stockage du constituant « X » et/ou des raisons de gestion améliorée de la logistique liée aux approvisionnements.

Ainsi, la présente invention concerne également dans un mode d'exécution particulier, l'utilisation des procédés/méthodes susmentionnés pour la préparation d'un produit cible comprenant un constituant « X », utilisation consistant à privilégier l'utilisation dudit constituant « X » parmi les « n » constituants du mélange produit cible, le procédé de préparation incluant une étape additionnelle consistant à sélectionner parmi les gammes de proportions respectives des constituants alimentant le mélangeur, la gamme de proportion ayant la concentration en constituant « X » la plus élevée.

REMPLACEMENT « X' »

Dans un autre mode d'exécution particulier, la présente invention consiste également en une méthode de sélection améliorée du choix en approvisionnement en constituant « X' » pour le remplacement du constituant « X » caractérisée en ce que cette méthode comprend les étapes suivantes

a. une étape d'analyse spectrale du constituant « X' » pour en déterminer une donnée spectrale caractérisante, par exemple un spectre et/ou une banque de données spectrales caractérisante,
b. une deuxième étape consistant en une étape de remplacement de la donnée spectrale caractérisante du constituant « X » par la donnée spectrale caractérisante du constituant « X' » dans le procédé M,
c. des étapes correspondant aux étapes 1 et 3 du procédé M,
d. l'étape 3 du procédé M des étapes précédentes permettant de prédire le taux d'utilisation potentiel du constituant « X' » dans le produit cible.

Cette méthode de sélection améliorée du choix en approvisionnement en constituant « X' » pour le remplacement du constituant « X » est particulièrement révolutionnaire car elle n'est plus basée uniquement sur des considérations économiques. En effet, l'analyse spectrale du nouveau constituant « X' » permettra à l'homme du métier de prendre en compte une multitude d'autres facteurs parmi lesquels nous citerons à titre d'exemple, les aspects environnementaux, des défauts en approvisionnement, etc...

A titre illustratif, la deuxième étape susmentionnée peut consister soit en une étape de remplacement du spectre du constituant « X » par le spectre du constituant « X' » dans la méthode M1, soit une étape de remplacement de la banque de données spectrales du constituant « X » par la banque de données spectrales du constituant « X' » dans la méthode M2.

Ainsi, la présente invention concerne également dans un mode d'exécution particulier, l'utilisation des procédés/méthodes susmentionnés pour la préparation d'un produit cible

    a. pour valider le remplacement d'un constituant « X » par un constituant « X' », et/ou
    b. prédire le taux d'utilisation potentiel du constituant « X' »

lors de la préparation d'un produit cible comprenant initialement un constituant « X », le procédé de préparation incluant

    a. une étape d'analyse spectrale du constituant « X' » pour en déterminer une donnée spectrale caractérisante, par exemple un spectre et/ou une banque de données spectrales caractérisante,
    b. une étape consistant en une étape de remplacement de la donnée spectrale caractérisante du constituant « X » par la donnée spectrale caractérisante du constituant « X' » dans le procédé,
    c. une étape de validation du remplacement d'un constituant « X » par un constituant « X' » et/ou de prédiction le taux d'utilisation potentiel du constituant « X' » qui permet de vérifier qu'il existe au moins une gamme de proportions respectives des nouveaux constituants nécessaires à la reconstitution d'une donnée spectrale du mélange appartenant au domaine spectral du produit cible.

Selon un mode d'exécution préféré de la présente invention, la méthode d'analyse spectrale utilisée est donc choisie parmi, par exemple la RMN, le Raman, l'IR, le NIR et/ou l'UV/Visible, de préférence par analyse spectrale topologique dans le proche infrarouge (« NIR »).

Selon un mode d'exécution préféré de la présente invention, le produit cible est un carburant. Parmi les constituants des carburants, nous citerons à titre illustratif les diesels, les gazoles oxygénés, les essences, les oxygénats (par exemple BOB pour « blend stock for oxygenate bending »), les esters d'acides gras (par exemple FAME), les esters d'huile végétale (par exemple les esters d'éthyle et/ou les esters de méthyle), les méthyl-ter-butyl-éther (MTBE), le méthyl-ter-amyl-éther (TAME), l'éthyl-ter-butyl-éther (ETBE), les huiles végétales hydrogénées (« HVO ») ou partiellement hydrogénées, l'ethanol, les bioéthanols, le méthanol, etc.... Ces carburants peuvent également contenir toute sorte d'additifs optionnels parmi lesquels nous citerons les pro-cétane et/ou pro-octane et/ou pro-heptane, les modificateurs de friction, les détergents, les antioxydants, les améliorants de tenue à froid, les améliorants de combustion, les agents anticorrosion et/ou leurs mélanges.

Ainsi, selon un mode d'exécution de la présente invention, le mélange de constituants de produits cibles peut se faire dans tout lieu approprié ; on citera à titre d'exemple tout complexe industriel comprenant des opérations de mélange de constituants pour la préparation d'un produit cible, par exemple une raffinerie, un complexe (pétrochimique, un dépôt pétrolier et/ou tout dispositif utilisant un produit constitué d'un mélange de constituants préparé par batch et/ou en ligne. La présente invention s'applique plus particulièrement aux terminaux ou toute installation de mélange post-raffinerie, de préférence de mélange de carburants.

L'analyse spectrale topologique dans le domaine du proche infrarouge (« NIR ») s'est avérée particulièrement efficace pour permettre la certification et la caractérisation d'un produit pétrolier cible et de ses constituants selon la présente invention.

Selon un mode d'exécution de la présente invention, on utilise dans l'étape 3, un programme informatique permettant de calculer les gammes de proportions respectives des dits constituants nécessaires à la reconstitution d'une donnée spectrale (donnée spectrale et/ou spectre et/ou banque de données spectrales) de mélange appartenant au domaine spectral de l'étape 1 à partir de donnée(s) spectrales(s) (donnée spectrale et/ou spectre et/ou banque de données spectrales) de l'étape 2. L'homme de l'art dispose de nombreux programmes informatiques qui permettent d'effectuer ces calculs. A titre purement illustratif et non restrictif, nous citerons ci-après les programmes informatiques basés sur des calculs mathématiques utilisant l'algorithme du Simplexe (tout autre algorithme de résolution des problèmes d'optimisation linéaire peut également être utilisé) et/ou les programmes informatiques basés sur des calculs mathématiques utilisant la méthode de Nelder-Mead (tout autre algorithme d'optimisation non-linéaire peut également être utilisé).

La caractérisation d'un produit selon la présente invention peut consister en une détermination et/ou une prédiction de toute caractéristique chimique, physique ou physico-chimique du dit produit et de ses constituants et/ou l'identification d'un type et/ou famille de constituants.

Le brevet EP0742900 de la Demanderesse constitue la référence du domaine en matière d'analyse spectrale topologique. Il y est décrit une méthode de détermination ou de prédiction d'une valeur Px, d'une propriété d'une matière X ou d'une propriété d'un produit résultant d'un procédé provenant de ladite matière ou du rendement dudit procédé, méthode qui consiste à mesurer l'absorption $D_i x$ de ladite matière à plus d'une longueur d'onde dans la région des 600 à 2 600 nm, à comparer les signaux indicateurs de ces absorptions ou leurs fonctions mathématiques avec des signaux indicateurs des absorptions Dim aux mêmes longueurs d'onde ou leurs fonctions mathématiques pour un certain nombre d'étalons S dans une banque pour lesquels on connaît ladite propriété ou rendement P, et à choisir dans la banque au moins un et de préférence au moins 2 étalons Sm ayant la propriété Pm, ledit étalon Sm ayant les valeurs moyennes les plus petites des valeurs absolues de la différence à chaque longueur d'onde i comprise entre le signal pour la matière et le signal pour l'étalon Sm en vue d'obtenir la valeur Px et à faire la moyenne desdites propriétés ou rendements Pm, lorsqu'on choisit plus d'un étalon Sm.

L'analyse spectrale topologique présente de nombreux avantages par rapport aux méthodes mathématiques régressives classiques. Les méthodes numériques décrites pour la modélisation des propriétés physico-chimiques de substances à base d'analyse spectrale sont de nature corrélative et impliquent des relations à caractère de régression entre la (ou les) propriété(s) étudiée(s). Parmi ces analyses à multiples variables on trouve la régression multilinéaire (MLR), la régression sur composant principal (PLR), la régression canonique et la régression des moindres carres partiels(PLS). Dans tous les cas, on cherche une relation entre la propriété et le spectre qui peut être linéaire mais qui est habituellement quadratique ou d'une forme algébrique supérieure comportant des coefficients de régression appliqués à chaque absorption. Hors, l'établissement de toute régression demande un étalonnage progressif, puisque l'approche est empirique et n'est pas soutenue par une théorie.

Ces techniques ont des inconvénients, dont le principal est la nécessité d'établir une corrélation forte entre le spectre et la propriété, et leur difficulté à traiter la synergie positive ou négative entre les composants contribuant à cette propriété. Par exemple, pour déterminer la composition chimique par exemple LINA (linéaire, isoparaffinique, naphténique, aromatique) dans une charge d'hydrocarbure alimentant un reformeur catalytique, on a décrit l'utilisation d'une technique PLS sur la base de spectres NIR. Le modèle marche bien sur l'ensemble d'étalonnage mais la réponse des modèles lorsqu'on ajoute des hydrocarbures purs, par exemple du cyclohexane, n'est pas satisfaisante, puisque le modèle prédit des variations en teneur d'isoparaffines et de naphtènes inverses de celles trouvées expérimentalement. De plus, il existe d'autres difficultés pratiques, principalement dues à la nécessité d'identifier des échantillons de familles ayant le même type de relation entre les spectres et les propriétés à modéliser. Ainsi, le modèle peut être limité, en particulier avec une relation non linéaire entre le spectre et la propriété. Surtout lorsqu'aux limites des données disponibles, la précision du modèle se réduit. La stabilité du modèle est également un problème, ainsi que la nécessité lors de l'addition de nouveaux étalons, de réaliser des révisions laborieuses pour obtenir le nouveau modèle, en particulier lorsqu'on ajuste a une nouvelle charge alimentant un procédé; ainsi le contrôle de 6 propriétés sur 4 produits sortant d'une unité de distillation demande 24 modèles, dont chacun doit être modifié pour chaque modification de la charge d'alimentation non comprise dans l'étalonnage. Un autre inconvénient majeur rencontré par ces techniques apparait lorsqu'un point à analyser se situe en dehors du modèle préalablement établi ; il faut alors générer une nouvelle base de données et un nouveau modèle par propriété ce qui rend ce type de technique non seulement peu réactive mais également nécessitant un nombre d'heures de travail beaucoup trop important.

Il faut noter que l'analyse spectrale topologique en tant que telle n'a pas réellement évolué depuis le brevet EP0742900 de la Demanderesse. Toutefois, la présente invention apporte également de nombreuses améliorations à la dite méthode d'analyse spectrale topologique. Les caractéristiques de cette méthode d'analyse spectrale topologique ainsi que ses améliorations et avantages seront décrits dans leurs détails dans la description qui va suivre ainsi que dans les exemples, figures et revendications. D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux figures 1 à 10 jointes en annexe et dans lesquelles :

- la figure 1 représente le spectre NIR d'un étalon,
- la figure 2 représente un exemple de banque de données spectrales A,
- la figure 3 représente un exemple de banque de données spectrales B (mise en évidence des longueurs d'onde polluantes),
- la figure 4 représente un exemple de banque de données spectrales améliorée A' (banque de données spectrales A dans laquelle on a supprimé les données spectrales correspondant aux longueurs d'onde polluantes),
- la figure 5 représente un exemple de banque de données spectrales élargie E (banque de données spectrales A ou A' dans laquelle on a ajouté des intergermes),
- la figure 6 représente un exemple de banque de données spectrales élargie EE (banque de données spectrales A et/ou E dans laquelle on a ajouté des extragermes),
- la figure 7 représente un exemple de banque de données spectrales élargie EEI (banque de données spectrales E et/ou EE dans laquelle on a ajouté des extragermes'),
- les figures 8 et 9 représentent respectivement un graphique et un tableau représentants des agrégats discriminants, et

- la figure 10 représente une banque de données spectrales du type de celle de la figure 5 dans laquelle ont été ajoutées les caractérisations mesurées des étalons et calculs des intergermes.

En particulier, toutes les approches chémométriques d'analyses spectrales de l'art antérieur requièrent l'établissement d'une banque de données spectrales constituée à partir d'un nombre initial très important d'échantillons et/ou étalons. Bien que l'art antérieur cite des constitutions de banque de données spectrales basées à partir d'au moins 60 ou au moins 100 échantillons et/ou étalons, tous les exemples décrivent des banques constituées d'un nombre d'échantillons nettement supérieurs. Ce nombre est encore plus grand dans les approches chémométriques utilisant les méthodes mathématiques régressives dont les banques de données sont constituées à partir de centaines voire milliers d'échantillons et/ou étalons. La présente invention, dans un mode particulier d'exécution, permet de s'affranchir de cette exigence antérieure ce qui ouvre un nombre considérable de nouvelles applications comme démontré ci-après.

Ainsi, dans un mode particulier d'exécution, dans un premier temps, le procédé selon la présente invention consiste en la préparation d'une banque de spectres et/ou de données spectrales des produits cibles et de leurs constituants, de préférence d'une banque de spectres et/ou de données spectrales élargie E pour un nombre limité de matières d'étalons disponibles (et représentant donc les produits cibles et/ou leurs constituants).

La présente invention s'applique donc plus particulièrement à la spectroscopie du proche infrarouge (NIR). En effet, la spectroscopie NIR présente de nombreux avantages par rapport à d'autres procédés d'analyse, par exemple, dans les raffineries, les sites pétrochimiques ou chimiques ainsi que tous les domaines où la caractérisation de produits chimiques, par exemple les hydrocarbures, en particulier les carburants, et elle peut recouvrir un grand nombre d'applications répétitives avec précision, avec rapidité et en ligne. De plus, la région des NIR entre 800 et 2500 nm contient la totalité des informations moléculaires sous forme de combinaisons et d'harmoniques de vibrations polyatomiques.

Dans une première étape, on effectue un type sélectionné d'analyse spectrale sur chacun des étalons (représentatifs du produit cible et/ou de ses constituants) et on procède au peuplement de la banque de spectres et/ou de données spectrales A en y enregistrant les spectres (par exemple sous forme numérisée ou digitalisée), de préférence les spectres NIR, à plusieurs longueurs d'onde (ou nombres d'onde), par exemple pour un nombre limité de matières d'étalons disponibles.

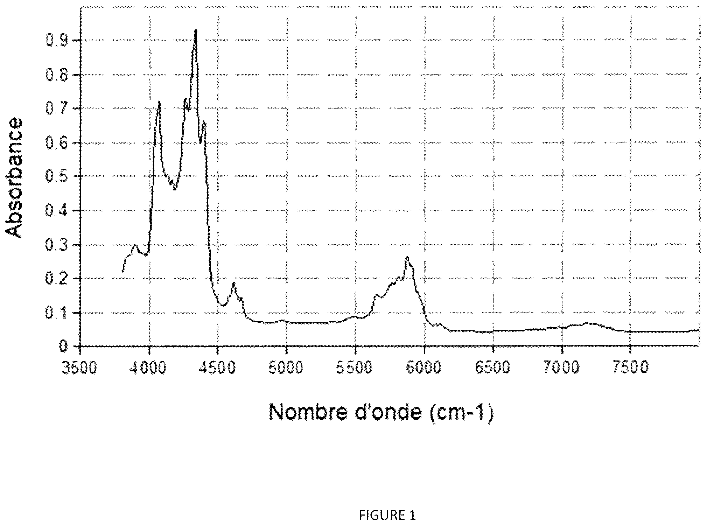

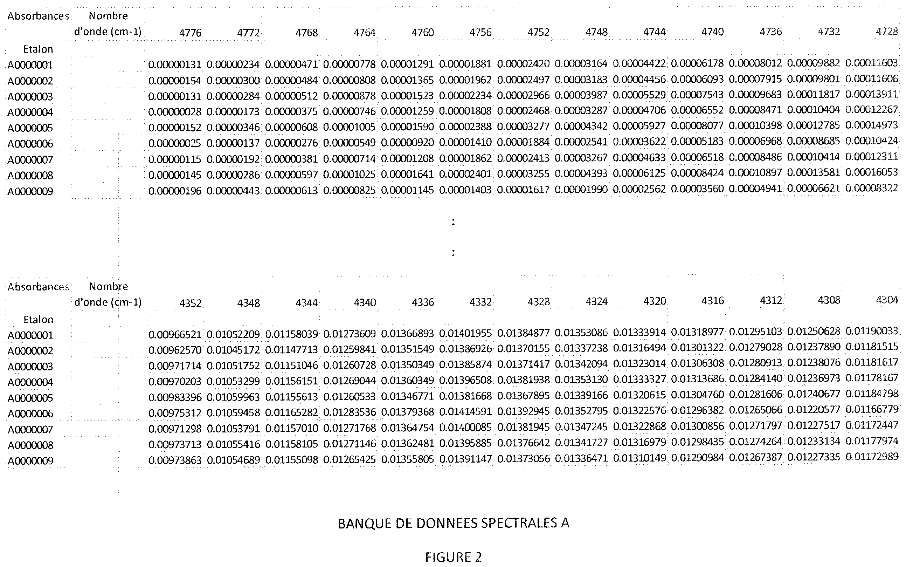

Un exemple de constitution et de représentation de cette banque de données spectrales initiale est décrit au moyen des figures 1 et 2.

La figure 1 représente le spectre NIR d'un étalon sur lequel on peut visualiser comme grandeur spectrale l'absorbance mesurée en fonction du nombre d'onde. Des spectres similaires sont donc également établis de manière identique pour chaque étalon. Dans le présent exemple de représentation, neuf étalons ont été analysés. A partir de ces spectres, on établit un tableau (la banque de données spectrales A) dont un exemple de représentation est donné dans la figure 2 pour un nombre limité de nombres d'onde.

Ainsi, dans le tableau de la figure 2 (qui correspond donc en une vue tronquée - on a représenté deux parties du tableau à des nombres d'onde sélectionnés différents), on peut apercevoir dans la colonne de gauche les références permettant d'identifier les neuf étalons et dans la première ligne la valeur des nombres d'onde ou gammes de nombres d'onde ; le contenu du tableau indique donc les valeurs des grandeurs spectrales (dans le cas présent, les absorbances) qui correspondent au couple « référence étalon / nombre d'onde ». Ces grandeurs spectrales peuvent être tous types de signaux caractérisant les spectres, par exemple les absorbances, transmittances, réflectances, etc... ; les absorbances ou densités optiques étant les signaux les plus communément utilisés. A titre d'exemple, on citera également comme signaux les dérivées des absorbances ou encore tout autre mesure résultant d'un autre type de traitement mathématique des dites absorbances.

Le nombre limité d'étalons disponibles est généralement dicté par le client et/ou l'utilisateur final, désireux d'utiliser des méthodes de contrôle réactives et fiables tout en limitant la nécessité de devoir disposer au préalable d'une grande quantité d'étalons et de devoir en effectuer une analyse selon des méthodes conventionnelles.

Une caractéristique du procédé selon la présente invention est qu'il permet donc de s'affranchir du besoin dicté par l'art antérieur de disposer d'un nombre très important d'étalons. Par exemple, la présente invention permet de caractériser un produit échantillon à partir d'un nombre d'étalons disponibles inférieur à 100, voire inférieur à 60 ou même inférieur à 50. Des résultats très probants ont même pu être obtenus au moyen de la présente invention à partir de moins de 40 étalons disponibles, voire moins de 30 ou même moins de 20. Un minimum de 10 étalons disponibles est toutefois préféré même si la présente invention a déjà été utilisée avec succès avec un minimum de 5 étalons disponibles.

Pour la présente invention, la description qui en est faite et les revendications ci-après, il est évident pour l'homme du métier que les spectres peuvent être réalisés en fonction des longueurs d'onde (et/ou gammes de longueurs d'onde) et/ou en fonction des nombres d'onde (et/ou gammes de nombres d'onde), car le nombre d'onde est représenté par l'inverse de la longueur d'onde.

Pour la présente invention, la description qui en est faite et les revendications ci-après, les étalons seront aussi bien qualifiés de « germes » [« G »], les deux termes étant interchangeables.

Une seconde étape optionnelle et préférée selon un mode d'exécution de la présente invention consiste ensuite en l'élimination des longueurs d'onde et/ou gammes de longueurs d'onde « polluantes » de la banque de données spectrales A. Cette étape consiste

1. à répéter au moins deux fois, de préférence au moins trois fois, plus préférentiellement au moins cinq fois la même analyse spectrale que celle effectuée lors de la première étape, et ceci sur au moins un des étalons disponibles, de préférence sur au moins deux ou même sur la totalité des dits étalons ;

2. à construire une banque de données spectrales B à partir des mesures effectuées au point 1 ci-dessus ;

3. à calculer pour chaque étalon sélectionné au point 1 ci-dessus et pour chaque longueur d'onde et/ou gamme de longueur d'onde (de la banque de données spectrales A) les écarts-type ($\sigma$) des mesures enregistrées dans la banque de données B ;

4. à identifier dans la banque de données B les longueurs d'onde et/ou gamme de longueur d'onde pour lesquelles l'écart-type est supérieur à une valeur prédéterminée ;

5. à supprimer de la banque de données spectrales A les mesures correspondant aux longueurs d'onde identifiées au point 4 ci-dessus.

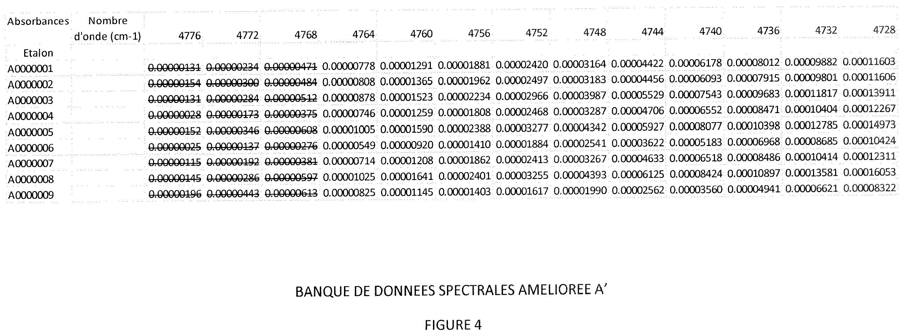

Ainsi, selon un mode d'exécution préféré de la présente invention, l'utilisation de la seconde étape ci-dessus permet l'obtention d'une banque de données spectrales améliorée A' ; un exemple de banque de données spectrales améliorée A' est représentée dans la figure 4.

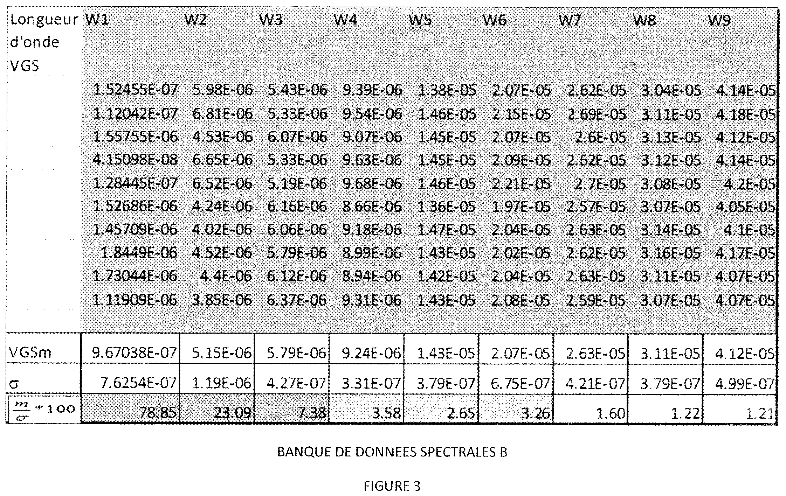

Un exemple de représentation de la banque de données spectrales B est illustré dans la figure 3 par un tableau.

On peut y voir que la même analyse spectrale a été répétée dix (10) fois sur le même échantillon et que les valeurs de grandeurs spectrales correspondantes ont été enregistrées dans le tableau. Les trois dernières lignes du tableau correspondent respectivement et successivement à

- la valeur de grandeur spectrale moyenne VGSmoyenne (« VGSm ») qui correspond à la somme des valeurs de grandeur spectrale divisée par le nombre (« n ») d'analyses effectuées (VGSm = [$\sum$ VGS]/n), avec n=10 dans la présente représentation ;
- l'écart-type (« $\sigma$ ») qui correspond à la différence entre VGSmax et VGSmin dans chaque colonne du tableau ;
- le ratio ($\sigma$/(VGSm/100)) dont la valeur (en pourcentage) est calculée en divisant l'écart-type par la valeur de la grandeur spectrale moyenne, le résultat étant multiplié par cent.

Ainsi, la dernière ligne du tableau permet d'identifier dans la banque de données B les longueurs d'onde et/ou gammes de longueurs d'onde pour lesquelles le ratio ($\sigma$/(VGSm/100)) est supérieur à une valeur prédéterminée. Selon un mode d'exécution de la présente invention, on identifie dans le tableau B les colonnes (les longueurs d'onde et/ou gammes de longueurs d'onde) pour lesquelles la valeur des ratios ($\sigma$/(VGSm/100)) est supérieure à 2% (de préférence supérieure à 1,5% ou même 1%) ; ensuite, on élimine de la banque de données A les dites colonnes, à savoir les valeurs de grandeurs spectrales correspondantes aux longueurs d'onde « polluantes ». Les colonnes correspondantes (c'est-à-dire celles dont la longueur d'onde et/ou gamme de longueurs d'onde sont identiques) seront ensuite éliminées de la base de données spectrales A. Il faut noter que dans les exemples ci-dessus, les tableaux A et B constituent des représentations qui n'ont pas de véritables relations entre elles ; il faut également noter que les tableaux A et B ont été tronqués de manière à en donner une représentation visuelle ; en réalité, les dits tableaux comprennent une multitude de colonnes représentant les longueurs d'onde et/ou gammes de longueurs d'onde extraites du spectre correspondant comme détaillé plus avant dans la description.

Un exemple de représentation de la banque de données spectrales améliorée A' est donc illustré dans la figure 4.

Une caractéristique essentielle du procédé selon un mode d'exécution de la présente invention consiste en ce que l'établissement de la banque de données spectrales améliorée A' n'a pas nécessité à ce stade de faire référence et/ou la moindre corrélation avec les propriétés chimiques et/ou physico-chimiques des étalons. En effet, cette deuxième étape en est totalement indépendante.

Une troisième étape consécutive préférée du procédé selon un mode d'exécution de la présente invention consiste en l'élargissement proprement dit de la banque de données spectrales A (ou de la banque de données spectrales améliorée A'). Cette étape consiste à générer des étalons synthétiques (également appelés « intergermes » [« IG »]) à partir des étalons disponibles et de leurs valeurs de grandeurs spectrales. Par exemple, pour générer ces IG on peut effectuer des combinaisons de plusieurs étalons disponibles de la première étape ci-dessus et peupler la banque de données spectrales A (ou la banque de données spectrales améliorée A') au moyen des dites combinaisons. Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons G. Selon un mode d'exécution préféré de la présente invention la dite combinaison consiste en un barycentre des valeurs de grandeurs spectrales (« VGS ») d'au moins deux étalons. On pourra par exemple effectuer ces combinaisons entre deux, trois ou un nombre supérieur des étalons disponibles de départ, de préférence entre tous les étalons disponibles de départ.

Un exemple de formule correspondante pour la génération d'un étalon synthétique (IG) à partir des étalons G (auquel correspondent les VGS) est

$$[\Sigma\ \text{Ri x VGSi}]\ /\ [\Sigma\ \text{Ri}]$$

pour i étant un nombre entier allant de 1 au nombre d'étalons G choisis pour cette combinaison et R étant un nombre réel tel que

$$[\Sigma\ \text{Ri}] > 0,$$

et

$$\left|[\Sigma\ \text{R*i}]\right| / [\Sigma\ \text{Ri}]\ <\ 0.3\ ,$$

de préférence < 0.15,

Et avec R* représentant uniquement les nombres réels négatifs.

Cette dernière formule peut aussi s'énoncer comme étant la valeur absolue de la somme des réels négatifs divisée par la somme de tous les réels.

Selon un mode d'exécution préféré de la présente invention, au moins un des Ri est un réel négatif (R*).

En procédant de la sorte, cela permet donc d'élargir au moyen d'étalons synthétiques (également appelés « intergermes » ou « IG ») la banque de données spectrales A (ou la banque de données spectrales améliorée A') et donc d'obtenir une banque de données spectrales élargie E.

Selon un mode d'exécution préféré de la présente invention, lorsque le nombre d'étalons de la banque de données spectrales A (ou A') vaut « N », le nombre d'intergermes IG est au moins supérieur à 1,5 N, de préférence supérieur à 2 N, plus préférentiellement supérieur à 5 N, voire supérieur à 10 N.

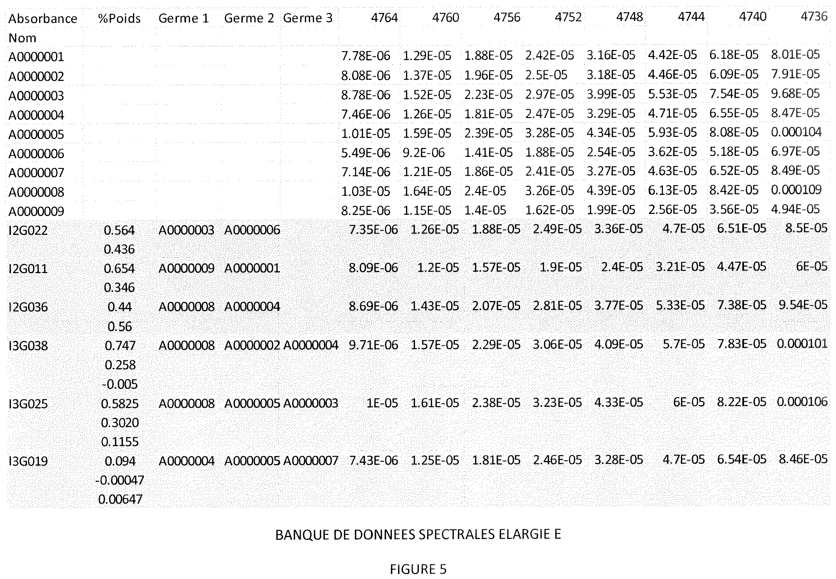

Un exemple de représentation de la banque de données spectrales élargie E est illustré dans la figure 5 par un tableau. On peut y voir que des étalons synthétiques (ou intergermes « IG ») ont été générés par combinaisons mathématiques et que les valeurs de grandeurs spectrales correspondantes ont été enregistrées dans le tableau E. On peut observer à titre d'exemple dans le tableau E (Figure 5) :

- six intergermes « IG » (I2G022, I2G011, I2G036, I3G038, I3G025 et I3G019;
- dans les colonnes 3 à 5, les germes utilisés pour générer chacun des dits intergermes ;
- dans la colonne 2, la pondération appliquée aux germes sélectionnés pour le calcul des VGS des intergermes (par exemple, pour le calcul de l'intergerme I2G036, on a appliqué une pondération de (0,44 fois le germe A0000008 + 0,56 fois le germe A0000004)).

Une caractéristique essentielle du procédé selon un mode d'exécution de la présente invention consiste en ce que l'établissement de la banque de données spectrales élargie E n'a pas nécessité à ce stade de faire référence et/ou la moindre corrélation avec les propriétés chimiques et/ou physico-chimiques des étalons. En effet, cette étape d'élargissement en est totalement indépendante.

Une quatrième étape additionnelle optionnelle et préférée selon un mode d'exécution de la présente invention consiste ensuite en un élargissement supplémentaire de la banque de données spectrales A ou de la banque de données spectrales élargie E au moyen d'un autre type d'étalons synthétiques que nous appellerons « extragermes » (« EG »). Cette étape est particulièrement pertinente lorsque le produit cible à analyser contient une pluralité de composés chimiques.

Elle consiste dans une première séquence à enregistrer les données spectrales d'au moins un spectre correspondant à un (ou plusieurs) des composés chimiques du produit cible (aussi appelé « Pôle(s) »). Ensuite, dans une seconde séquence, on procède à un élargissement additionnel de la banque de données spectrales en utilisant le(s) dit Pôle(s) et en les combinant avec les germes « G » (on effectue donc une combinaison de leurs valeurs de grandeur spectrale VGS).

Cette seconde séquence consiste à générer des étalons synthétiques (également appelés « extragermes »

[« EG »]) à partir du/des Pôle(s) et des étalons disponibles et de leurs valeurs de grandeurs spectrales. Par exemple, pour générer ces EG on peut effectuer des combinaisons de(s) Pôle(s) et de plusieurs étalons disponibles de la première étape ci-dessus et peupler la banque de données spectrales A et/ou E au moyen des dites combinaisons. Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons G et du/des Pôle(s). Selon un mode d'exécution préféré de la présente invention la dite combinaison consiste en un barycentre des valeurs de grandeurs spectrales (« VGS ») des étalons G sélectionnés et du/des Pôle(s). On pourra par exemple effectuer ces combinaisons entre au moins un Pôle et un, deux, trois ou un nombre supérieur des étalons disponibles de départ, de préférence avec tous les étalons disponibles de départ. Ces combinaisons s'effectueront de préférence avec tous les Pôles disponibles, de préférence avec tous les Pôles correspondant à tous les composés chimiques constituant le produit cible.

Un exemple de formule correspondante pour la génération d'un étalon synthétique de type EG à partir de Pôle(s) et d'étalons G (auxquels correspondent les VGS) est $[\sum R_i \times VGS_i + \sum R_j \times VGS_j] / [\sum R_i + \sum R_j]$

pour i étant un nombre entier allant de 1 au nombre d'étalons G choisis pour cette combinaison, j étant un nombre entier allant de 1 au nombre de Pôle(s) choisis pour cette combinaison

et R étant un nombre réel tel que

$$[\sum R_i + \sum R_j] > 0,$$

et

$$\left| [\sum R*i] \right| / [\sum R_i + \sum R_j] < 0.3 \text{ , de préférence} < 0.15, \qquad (I)$$

avec R* représentant uniquement les nombres réels négatifs,

et, de préférence, chaque Rj doit être tel que le rapport

Rj / [$\sum R_i + \sum R_j$] soit toujours compris entre l'opposé de la teneur minimale et la teneur maximale en pourcentage en poids du Pôles j dans le produit cible.

La formule (I) ci-dessus peut aussi s'énoncer comme étant la valeur absolue de la somme des réels négatifs « i » divisée par la somme de tous les réels. Selon un mode d'exécution préféré de la présente invention, au moins un des Ri est un réel négatif (R*).

En procédant de la sorte, cela permet donc d'élargir au moyen d'étalons synthétiques « EG » (« extragermes ») la banque de données spectrales A et/ou E et donc d'obtenir une banque de données spectrales élargie EE. De manière optionnelle, les dits Pôles et leurs VGS peuvent aussi être intégrés dans la banque de données spectrales EE mais ceci ne constitue pas un mode d'exécution préféré selon la présente invention.

Selon un mode d'exécution préféré de la présente invention, lorsque le nombre d'étalons de la banque de données spectrales A (ou A') vaut « N » et le nombre de « Pôles » vaut « M » , le nombre d'extragermes « EG » est au moins supérieur à NxM , de préférence supérieur à 1,5 NxM, de préférence supérieur à 2 NxM.

Selon un mode d'exécution de la présente invention, le nombre de pôles est inférieur à 15, par exemple inférieur à 10.

Selon un mode d'exécution de la présente invention, le nombre de pôles est inférieur à 0,2 fois le nombre d'étalons, par exemple inférieur à 0,1 fois le nombre d'étalons.

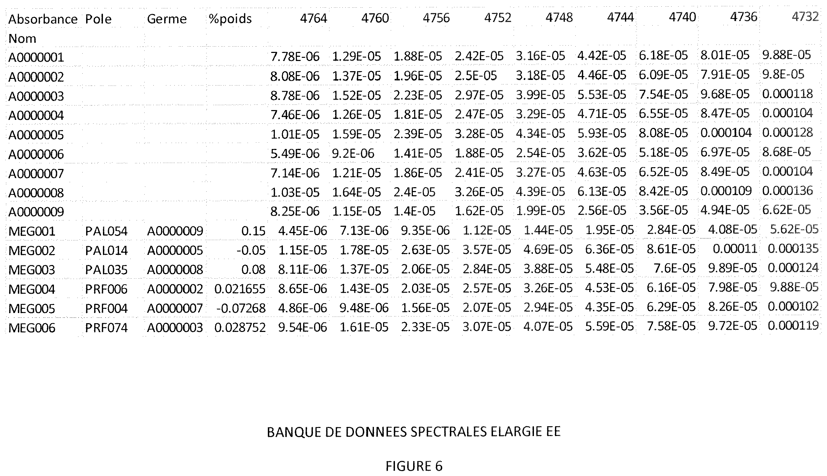

Un exemple de représentation de la banque de données spectrales élargie EE est illustré dans la figure 6 par le tableau EE. On peut y voir que les « Pôles » ainsi que la génération des étalons synthétiques « EG » (extragermes) par combinaisons mathématiques et les valeurs de grandeurs spectrales correspondantes ont été enregistrées dans le tableau. On peut observer à titre d'exemple dans le tableau EE (Figure 6) :

- six extragermes « EG » (MEG001 à MEG006);
- dans la colonne 2 (« Pôle »), la référence des pôles utilisés (par exemple, le Pôle PAL054 est un type particulier d'alkylat utilisé dans la composition d'essences constituant les étalons de la banque de données) ;
- dans la colonne 3, la référence du germe utilisé pour générer chacun des dits extragermes ;
- dans la colonne 4, la pondération appliquée aux Pôles (X)- la pondération appliquée aux germes étant donc de (1-X). Par exemple, pour le calcul de l'extragerme MEG001, on a appliqué une pondération de (0,15 fois le Pôle PAL054 + 0,85 fois le germe A0000009).

Une caractéristique essentielle du procédé selon un mode d'exécution de la présente invention consiste en ce que l'établissement de la banque de données spectrales élargie EE n'a pas nécessité à ce stade de faire référence et/ou la moindre corrélation avec les propriétés chimiques et/ou physico-chimiques des étalons. En effet, cette étape d'élargissement en est totalement indépendante.

Une cinquième étape additionnelle optionnelle et préférée selon la présente invention consiste également en un élargissement supplémentaire de la banque de données spectrales élargie E et/ou EE au moyen d'un autre type d'étalons synthétiques que nous appellerons

« extragermes' » (« EG' »). Cette étape est à nouveau particulièrement pertinente lorsque le produit cible à analyser contient une pluralité de composés chimiques.

Elle consiste dans une première séquence à enregistrer les données spectrales d'au moins un spectre correspondant à un (ou plusieurs) des composés chimiques du produit cible (aussi appelé « Pôle(s) »).

Ensuite, dans une seconde séquence, on procède à un élargissement additionnel de la banque de données spectrales E ou EE en utilisant le(s) dit(s) Pôle(s) et en les combinant avec les intergermes « IG » (combinaison de leurs VGS).

Cette seconde séquence consiste à générer des étalons synthétiques (également appelés « extragermes' » [« EG' »]) à partir du/des Pôle(s) et des étalons « intergermes » « IG » (et optionnellement des germes « G ») et de leurs valeurs de grandeurs spectrales. Par exemple, pour générer ces EG' on peut effectuer des combinaisons de(s) Pôle(s) et de plusieurs intergermes « IG » de la troisième étape ci-dessus (et optionnellement de germes « G » de la première étape) et peupler la banque de données spectrales E et/ou EE au moyen des dites combinaisons.

Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons synthétiques (intergermes) « IG » et du/des Pôle(s) (et optionnellement des germes « G »).

Selon un mode d'exécution préféré de la présente invention la dite combinaison consiste en un barycentre des valeurs de grandeurs spectrales (« VGS ») des intergermes IG et du/des Pôle(s)) (et optionnellement des germes « G »). On pourra par exemple effectuer ces combinaisons entre au moins un Pôle et un, deux, trois ou un nombre supérieur des « IG » de la troisième étape, de préférence avec tous les « IG » ; et optionnellement avec au moins un des germes « G », de préférence avec tous les germes « G ». Ces combinaisons s'effectueront de préférence avec tous les Pôles disponibles, de préférence avec tous les Pôles correspondant à tous les composés chimiques constituant le produit cible.

Un exemple de formule correspondante pour la génération d'un étalon synthétique de type EG' à partir de Pôle(s) et d'étalons synthétiques IG (auxquels correspondent les VGS) est

$[\Sigma\, R_i \times VGS_i + \Sigma\, R_j \times VGS_j + \Sigma\, R_k \times VGS_k] / [\Sigma\, R_i + \Sigma\, R_j + \Sigma\, R_k]$ pour k étant un nombre entier allant de 1 au nombre d'étalons synthétiques IG choisis pour cette combinaison, i étant un nombre entier allant de 0 (de préférence 1) au nombre d'étalons G choisis pour cette combinaison, j étant un nombre entier allant de 1 au nombre de Pôle(s) choisis pour cette combinaison et R étant un nombre réel tel que

$$[\Sigma\, R_i + \Sigma\, R_j + \Sigma\, R_k] > 0,$$

et

$$|[\Sigma\, R*_i] + [\Sigma\, R*_k]\,| / [\Sigma\, R_i + \Sigma\, R_j + \Sigma\, R_k] \quad < \quad 0.3 \;, \text{ de préférence} < 0.15, \qquad (II)$$

avec $R_k$ étant de préférence toujours positif,

avec R* représentant uniquement les nombres réels négatifs,

ET de préférence chaque $R_j$ doit être tel que le rapport

$R_j / [\Sigma\, R_i + \Sigma\, R_j + \Sigma\, R_k]$ soit toujours compris entre l'opposé de la teneur minimale et la teneur maximale en pourcentage en poids du Pôles j dans le produit cible. La formule (II) ci-dessus peut aussi s'énoncer comme étant la valeur absolue de la somme des réels négatifs « i » divisée par la somme de tous les réels. Selon un mode d'exécution préféré de la présente invention, au moins un des $R_i$ est un réel négatif (R*). En procédant de la sorte, cela permet donc d'élargir au moyen de d'étalons synthétiques « EG' » (« extragermes' ») la banque de données spectrales E et/ou EE et donc d'obtenir une banque de données spectrales élargie EEI. De manière optionnelle, les dits Pôles et leurs VGS peuvent aussi être intégrés dans la banque de données spectrales E mais ceci ne constitue pas un mode d'exécution préféré selon la présente invention.

Selon un mode d'exécution préféré de la présente invention, lorsque le nombre d'étalons synthétiques IG de la banque de données spectrales E vaut « Z » et le nombre de « Pôles » vaut « M », le nombre d'extragermes' « EG' » est au moins supérieur à ZxM , de préférence supérieur à 1,5 ZxM, de préférence supérieur à 2 ZxM. Selon un autre mode d'exécution préféré de la présente invention, lorsque le nombre d'étalons synthétiques IG de la banque de données spectrales E vaut « Z », le nombre de germes G vaut N et le nombre de « Pôles » vaut « M » , le nombre d'extragermes' « EG' » est au moins supérieur à ZxMxN , de préférence supérieur à 1,5 ZxMxN, de préférence supérieur à 2 ZxMxN.

Selon un mode d'exécution de la présente invention, le nombre de pôles est inférieur à 15, par exemple inférieur à 10.

Selon un mode d'exécution de la présente invention, le nombre de pôles est inférieur à 0,2 fois le nombre d'étalons, par exemple inférieur à 0,1 fois le nombre d'étalons.

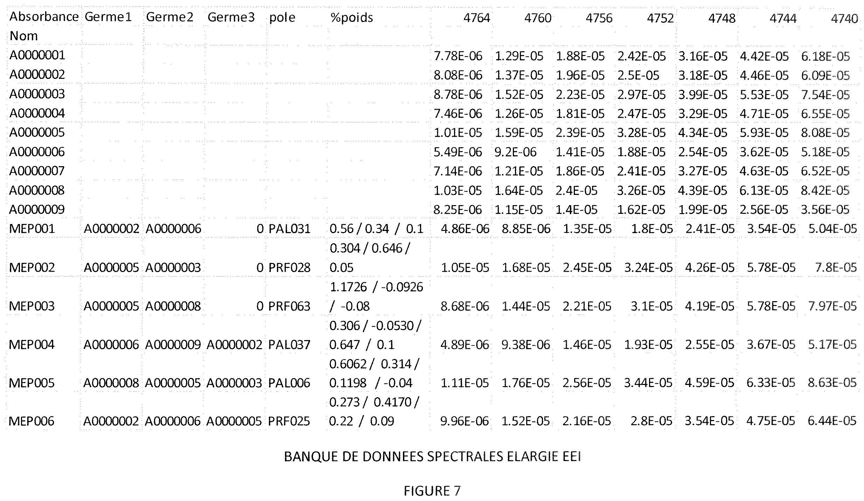

Un exemple de représentation de la banque de données spectrales élargie EEI est illustré dans la figure 7 par un tableau. On peut y voir les « Pôles » ainsi que la génération des étalons synthétiques « EG' » (extragermes') par combinaisons mathématiques et que les valeurs de grandeurs spectrales correspondantes ont été enregistrées dans le tableau. On peut observer à titre d'exemple dans le tableau EEI (Figure 7) :

- six extragermes' « EG' » (MEP001 à MEP006);
- dans la colonne 5 (« Pôle »), la référence des pôles utilisés (par exemple, le Pôle PAL037 est un type particulier d'alkylat utilisé dans la composition d'essences constituant les étalons de la banque de données) ;
- dans les colonnes 2 à 4, la référence des intergermes (combinaisons de germes) utilisé pour générer chacun des dits extragermes ;
- dans la colonne 6, la pondération appliquée. Par exemple, pour le calcul de l'extragerme MEP004, on a appliqué une pondération de

[0,9 fois un intergerme (correspondant à 0,306 fois le germe A00000061 - 0,0530 fois le germe A0000009 + 0,647 fois le germe A0000002) + 0,1 fois le pôle PAL037].

Une caractéristique essentielle du procédé selon un mode d'exécution de la présente invention consiste en ce que l'établissement de la banque de données spectrales élargie EEI n'a pas nécessité à ce stade de faire référence et/ou la moindre corrélation avec les propriétés chimiques et/ou physico-chimiques des étalons. En effet, cette étape d'élargissement en est totalement indépendante.

La présente divulgation concerne donc également une méthode de génération et d'optimisation d'une banque de données spectrales (utilisée préférentiellement dans les étapes 1 et 2 de la méthode M2 de préparation d'un produit pétrolier cible susmentionnée) pouvant servir dans un procédé de caractérisation d'un produit cible et/ou de ses constituants par analyse spectrale topologique à partir d'un nombre limité d'étalons disponibles, méthode consistant dans une première étape

- à effectuer la même analyse spectrale sur les dits étalons, et
- à constituer à partir des spectres obtenus une banque de données spectrales A à plusieurs longueurs d'onde et/ou gammes de longueurs d'onde,

caractérisée dans une seconde étape optionnelle en ce qu'on élimine de la banque de données spectrales A les longueurs d'onde et/ou gammes de longueurs d'onde « polluantes » de la dite banque de données spectrales A, seconde étape consistant

1. à répéter au moins deux fois, de préférence au moins trois fois, plus préférentiellement au moins cinq fois la même analyse spectrale que celle effectuée lors de la première étape, et ceci sur au moins un des étalons disponibles, de préférence sur au moins deux ou même sur la totalité des dits étalons disponibles ;
2. à construire une banque de données spectrales B à partir des mesures effectuées au point 1 ci-dessus ;
3. à calculer pour chaque étalon sélectionné au point 1 ci-dessus et pour chaque longueur d'onde et/ou gamme de longueur d'onde (de la banque de données spectrales A) les écarts-type ($\sigma$) des mesures enregistrées dans la banque de données B;
4. à identifier dans la banque de données B les longueurs d'onde et/ou gamme de longueur d'onde pour lesquelles l'écart-type est supérieur à une valeur prédéterminée ; et
5. à supprimer de la banque de données spectrales A les mesures correspondant aux longueurs d'onde identifiées au point 4 ci-dessus et obtenir ainsi une banque de données spectrales améliorée A',

et également caractérisée par une troisième étape consécutive préférée qui consiste en l'élargissement de la banque de données spectrales A (ou de la banque de données spectrales améliorée A'), étape consistant à effectuer des combinaisons de plusieurs étalons de la première étape et à peupler la banque de données spectrales A (ou la banque de données spectrales améliorée A') au moyen des dites combinaisons (appelées étalons synthétiques ou intergermes « IG ») et obtenir ainsi une banque de données spectrales élargie E,

et également caractérisée par une quatrième étape consécutive optionnelle qui consiste en l'élargissement de la banque de données spectrales E, étape consistant dans une première séquence à rajouter à la banque de données spectrales élargie E au moins un spectre correspondant à au moins un des composés chimiques (ou plusieurs) du produit cible (aussi appelé « Pôle(s) ») et dans une seconde séquence à effectuer des combinaisons mathématiques du(des) dit(s) Pôle(s) avec au moins un étalon G de la première étape et/ou au moins un des étalons IG de la troisième étape et à peupler la banque de données spectrales E au moyen des dites combinaisons (appelées respectivement soit étalons synthétiques extragermes « EG », soit étalons synthétiques extragermes' « EG' ») et obtenir ainsi une banque de données spectrales élargie EE (ou EEI).

Apres avoir constitué la banque de données spectrales élargie conformément à la méthodologie développée ci-dessus, il est possible d'utiliser toute sorte d'analyse mathématique conventionnelle pour caractériser un échantillon à partir de la banque de données spectrales élargie.

Selon un mode d'exécution préféré de la présente invention, avant cette caractérisation, une étape intermédiaire additionnelle consiste ensuite à définir une méthode de discrimination efficace permettant de mettre en évidence des sous-groupes homogènes de produits qui obéissent préférentiellement aux mêmes types de liaisons propriétés-spectres par suite d'une forte analogie de structure moléculaire.

Les méthodes de discrimination peuvent être exclusivement basées sur des techniques d'analyse mathématiques (par exemple, des analyses factorielles et/ou des analyses en composantes principales). Bien que certaines de ces méthodes mathématiques puissent s'avérer utiles, la présente invention utilise de préférence encore au moins une étape empirique pour effectuer ce type de discrimination, étape empirique basée sur une analyse visuelle des spectres des étalons et/ou des pôles susmentionnés ; bien que ceci ne constitue pas un mode d'exécution préféré selon la présente invention, cette analyse visuelle pourrait également se faire sur les spectres reconstitués (à partir de leurs VGS calculées) des intergermes et/ou extragermes. Cette étape empirique permet ainsi de mettre en évidence des différences infimes entre les spectres en question, différences qui après vérification peuvent s'avérer synonymes de l'existence de sous-groupes homogènes de produits même si on pouvait penser à l'origine que l'ensemble de la population des produits était homogène. Cette technique de discrimination permet ainsi de mettre en évidence des différences entre les produits alors même que l'utilisateur final n'en avait pas encore la connaissance.

Pour rappel, une caractéristique essentielle du procédé d'établissement de la banque de données spectrales élargie selon un mode préféré d'exécution de l'invention susmentionnée est qu'il n'a pas été nécessaire de faire référence et/ou la moindre corrélation avec les propriétés chimiques et/ou physico-chimiques des étalons. Selon un mode d'exécution préféré de la présente invention, il en est exactement de même pour l'étape de discrimination décrite ici.

AGREGATS

Ainsi, selon un mode d'exécution de la présente invention, l'étape de discrimination consiste ensuite à définir, à partir de la banque de données spectrales (de préférence élargie), des agrégats (de préférence au moins deux agrégats), des espaces à n dimensions représentant les combinaisons des dits agrégats (de préférence des plans - ou espaces à deux dimensions - représentant des couples d'agrégats), et des boites spectrales correspondantes. Selon un mode d'exécution préféré de la présente invention, ces agrégats et/ou ces espaces à n dimensions représentant des combinaisons des dits agrégats et/ou ces boites spectrales définissent le domaine spectral du produit cible et donc le fait que le mélange final soit conforme à un ensemble de spécifications significatives du produit cible.

Selon un mode d'exécution de la présente invention, la méthode de discrimination comprend également au moins deux caractéristiques préférées particulières :

1. le fait que la dite méthode implique une phase d'itération lors de laquelle on vérifie l'efficacité de la boite spectrale et donc la pertinence des agrégats sélectionnés ; et
2. le fait que les agrégats sont bâtis à partir d'au moins une analyse visuelle de l'allure des spectres qui permet ensuite de bâtir les équations des agrégats en fonction des valeurs de grandeurs spectrales VGS.

Les agrégats sont donc définis comme des fonctions mathématiques des valeurs de grandeurs spectrales de la banque de données spectrales élargie permettant de regrouper et/ou discriminer et/ou séparer des familles de produits au sein de la banque de données spectrales élargie.

Ces agrégats peuvent donc être représentés de manière générique par la fonction Agg = f (VGSi).

Selon un mode d'exécution préféré de la présente invention, la dite fonction répond aux équations

de type $\dfrac{\sum_{k=1}^{n}\sum_{i=1}^{p} ai\, Wi^{\propto} Wk^{\beta}}{\sum_{i=1}^{q} ai\, Wi^{\propto}}$ ou de préférence de type $\dfrac{\sum_{i=1}^{p} ai\, Wi^{\propto}}{\sum_{i=1}^{q} ai\, Wi^{\propto}}$ dans lesquelles

- W représentent les valeurs de grandeurs spectrales VGS discriminantes,
- a sont des réels positifs,
- p et q représentent la sélection des VGS aux longueurs d'ondes et/ou gammes de longueurs d'onde pertinentes pour l'étape de discrimination, et
- $\propto$ et $\beta$ sont des exposants compris entre 1/3 et 3.

En ce qui concerne la phase d'itération lors de laquelle on vérifie l'efficacité de la boite spectrale et donc la pertinence des agrégats sélectionnés, il suffit de rajouter à la banque de données spectrales préétablie des colonnes représentant les équations des agrégats discriminants, de calculer la valeur des dits agrégats pour chacun des étalons et/ou intergermes et/ou extragermes et/ou pôles de la banque de données spectrales, d'en faire les représentations graphiques (de préférence dans des espaces à deux dimensions par paire d'agrégats), et de visualiser ainsi si la discrimination a bien conduit à la mise en évidence de sous-groupes homogènes de produits. Cette étape de discrimination permet donc de diviser la banque de données spectrales en plusieurs (au moins deux) familles distinctes (sous-groupes homogènes de produits), de préférence au moins trois familles distinctes.

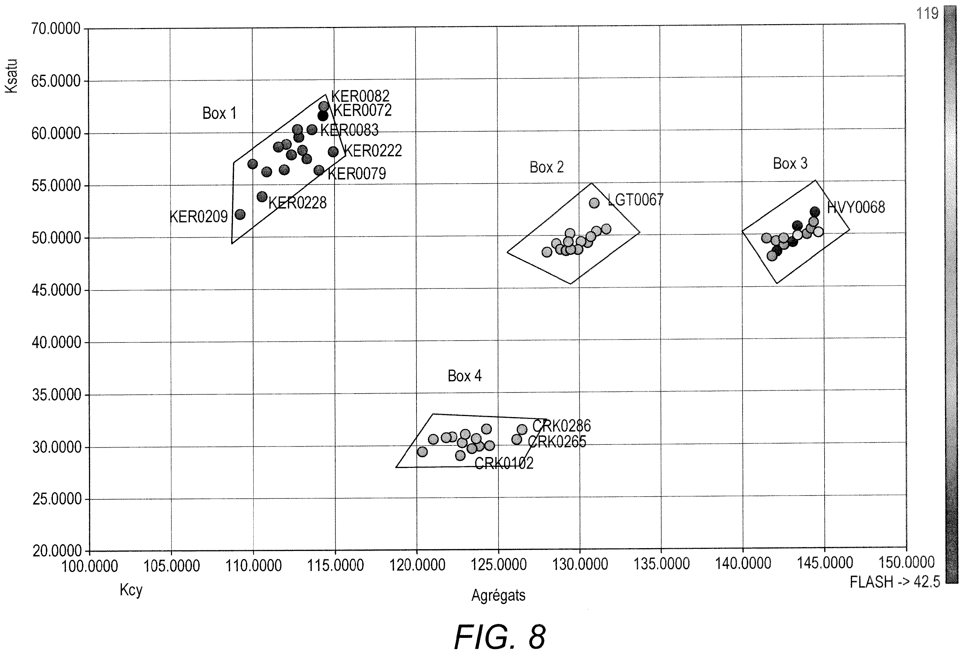

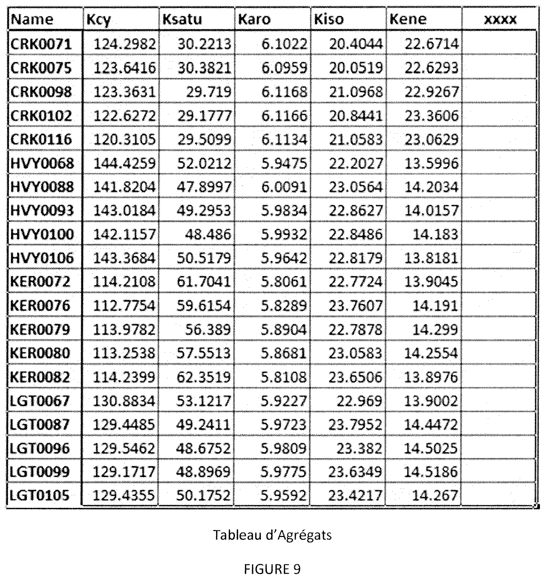

A titre d'exemple, les figures 8 et 9 représentent respectivement

- un graphique dont les axes abscisses/ordonnées correspondent à deux agrégats discriminants, et
- un tableau de valeurs correspondantes dont les colonnes représentent plusieurs agrégats discriminants dont les deux premiers ont servi à la constitution du graphique (figure 8).

Ces figures expliquent clairement comment on parvient à mettre en évidence plusieurs sous-groupes homogènes de produits ; ce qui permet la sélection du domaine spectral du produit cible.

La présente invention concerne donc également un procédé de caractérisation d'un produit par analyse spectrale topologique.

La caractérisation d'un produit selon la présente invention peut consister en une détermination et/ou une prédiction de toute caractéristique chimique, physique ou physico-chimique du dit produit.

Selon un mode d'exécution particulier de la présente invention, la première étape a donc été caractérisée par l'établissement d'une banque de données spectrales, de préférence une banque de données spectrales élargies comme décrite dans la présente description.

Comme déjà indiqué ci-dessus, les représentations graphiques des banques de données (tableaux) dans les figures annexées constituent des vues tronquées car en réalité les dites banques de données comprennent une multitude de colonnes représentant les longueurs d'onde et/ou gammes de longueurs d'onde (ou à titre équivalent, les nombres d'onde ou gamme de nombres d'onde) extraites des spectres correspondants.

Selon un mode d'exécution de la présente invention, le nombre de longueurs d'onde choisi peut être de 2 à 1000, par exemple de 5 à 200 ou de 40 à 80.

Les longueurs d'onde choisies peuvent être à des intervalles réguliers tels que 1 à 50 nm ou tous les 10 à 50 nm ou tous les 15 à 35 nm ou tous les 1 à 5 nm ou tous les nanomètres ; ou elles peuvent être à des intervalles irréguliers, par exemple à des intervalles de 1 à 200 nm, par exemple de 1 à 100 ou de 1 à 50 en particulier de 2 à 50 ou de 4 à 50 ou de 10 à 60 nm, qui peuvent être choisis ou aléatoires en raison d'une variation de la forme de la courbe spectrale à cette longueur d'onde par exemple, un pic, une vallée ou un épaulement ou bien choisis avec des critères chimiques ou statistiques tels que l'analyse factorielle. Les longueurs d'onde peuvent être dans la région des 600 à 20000 nm, par exemple de 625 à 2600 nm, par exemple de 800 à 2600 nm, en particulier de 1500 à 2600 ou de 2000 à 2550 nm. Les nombres d'ondes peuvent être dans la région des 16600 à 500, par exemple de 16000 à 3840 cm-1, par exemple de 12500 à 3840 cm-1 en particulier de 6660 à 3840 ou de 5000 à 3900 cm-1; les fréquences correspondantes en Hertz peuvent être obtenues en multipliant ces longueurs d'onde par 3x10(exp)10 cm/s.

Avant de pouvoir déterminer et/ou prédire une propriété d'un échantillon, il faut bien évidemment mesurer les valeurs de la dite propriété pour les étalons et, optionnellement, pour les pôles. Ainsi, selon un mode d'exécution de la présente invention, les propriétés chimiques, physiques et/ou physico-chimiques des étalons (et optionnellement des pôles) sont déterminées au moyen de techniques d'analyses conventionnelles. A titre d'exemple non limitatif des techniques d'analyse conventionnelle, on citera la chromatographie en phase gazeuse pour les compositions chimiques. Bien qu'il aille de soi que les étalons soient choisis pour couvrir la gamme dans laquelle on doit utiliser la méthode, la présente invention permet dans un mode d'exécution préféré de travailler avec un nombre limité d'étalons grâce à la méthodologie d'élargissement de la banque de données spectrales susmentionnée.

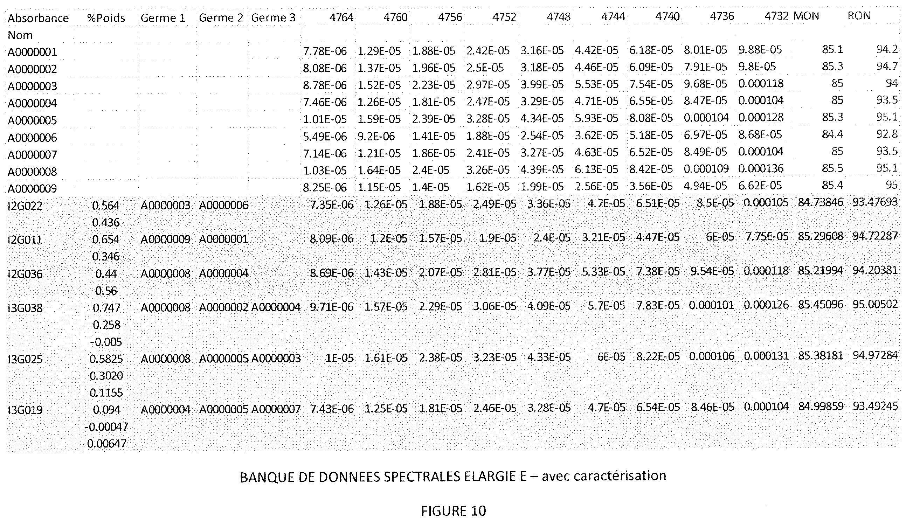

Ainsi, dans un mode d'exécution préféré de la présente invention, on rajoute à la banque de données spectrales les valeurs des propriétés désirées mesurées pour les dits étalons (et optionnellement des pôles); lorsque la banque de données spectrales est élargie, on calcule ensuite les valeurs des dites propriétés pour les étalons synthétiques intergermes (et optionnellement pour les extragermes) à partir des formules ayant servi à générer ces étalons synthétiques ; ce calcul se fait simplement en remplaçant les valeurs de grandeurs spectrales VGS par les valeurs mesurées des dites propriétés des étalons (et optionnellement des pôles) utilisés dans les formules (et optionnellement, pour les extragermes, par les valeurs déjà calculées pour les intergermes). On aboutit ainsi à une banque de données spectrales constituée d'un nombre de points (étalons et optionnellement intergermes, pôles et extragermes) auxquels sont associés les propriétés désirées (mesurées et calculées). Un exemple de réalisation (vue tronquée) est donné dans la figure 10.

Il s'agit à titre d'illustration d'une banque de données spectrales élargie E constituée d'étalons (A) et d'intergermes (IG). Le tableau a été complété au moyen des caractéristiques des produits cibles recherchées, à savoir les valeurs RON et MON (l'indice d'octane de recherche (RON) et l'indice d'octane de moteur (MON)). Ces caractéristiques ont donc été mesurées pour les étalons et calculées pour les intergermes.

Dans la description de EP0742900, on compare ensuite les signaux, par exemple les absorptions (ou leurs dérivées) pour l'échantillon inconnu, avec des signaux, par exemple les absorptions (ou leurs dérivées) à la même longueur d'onde des étalons, et on choisit les étalons ayant les différences les plus petites. Ensuite, on fait la moyenne des propriétés de ces étalons choisis pour déterminer la propriété de l'échantillon inconnu. On reconstitue donc un spectre calculé du produit cible auquel correspond la caractéristique (propriété) ainsi calculée.

Selon un mode d'exécution préféré de la présente invention, cette comparaison de signaux ne s'effectue donc pas sur l'entièreté de la banque de données spectrales mais uniquement sur la partie de banque de données spectrales représentative du sous-groupe homogène auquel l'échantillon appartient. C'est en utilisant de préférence la méthode de discrimination susmentionnée (agrégats discriminants) que cette partie de banque de données spectrales est définie.

Ensuite, on compare les signaux, par exemple les absorptions (ou leurs dérivées ou toute autre valeur de grandeur spectrale) pour l'échantillon inconnu (produit cible), avec les mêmes signaux et à la même longueur d'onde des étalons et/ou intergermes et/ou extragermes et/ou pôles appartenant au même sous-groupe homogène, et on choisit dans la banque de données spectrales les étalons et/ou intergermes et/ou extragermes et/ou pôles ayant les différences les plus petites.

Quelle que soit la méthode utilisée, nous appellerons par la suite les points proches du produit cible les points « proches voisins ». Ensuite, on peut par exemple faire la moyenne des propriétés de ces étalons et/ou intergermes et/ou extragermes et/ou pôles choisis pour déterminer la caractéristique recherchée (propriété) de l'échantillon inconnu.

Selon un mode d'exécution particulier de la présente invention, les proches voisins choisis sont ceux ayant les plus petites valeurs moyennes de la différence absolue à chaque longueur d'onde i entre la valeur de grandeur spectrale (représentée à titre d'exemple par l'absorbance ou une dérivée de celle-ci) Wix pour le produit cible (échantillon / produit inconnu) et le signal correspondant Wim pour le proche voisin. Les moyennes peuvent se rapporter par exemple à la valeur moyenne de Wix-Wim (quel que soit son signe, à savoir une différence absolue), ou de (Wix-Wim)exp2. Pour chaque proche voisin dans la banque de données spectrales pour le type de produit en question, on trouve la différence moyenne telle que décrite et on choisit le proche voisin ayant les différences moyennes les plus petites, à savoir au moins 1 mais de préférence 2, jusqu'à 1000 des plus petites par exemple 2 à 100 ou 2 à 20 mais en particulier 2 à 10 et surtout 2 à 6 des plus petites. Cette sélection des plus proches voisins peut être effectuée selon toute méthode connue, par exemple on peut utiliser de manière avantageuse les méthodes décrites dans la description du brevet EP0742900 (par exemple en déterminant l'indice de proximité).

Selon un mode d'exécution particulier de la présente invention, le nombre de proches voisins peut être égal à 1, de préférence supérieur ou égal à 2, encore de préférence supérieur ou égal à 3.

Selon un mode d'exécution de la présente invention, le nombre de proches voisins est inférieur ou égal à 50, par exemple inférieur ou égal à 20, voir même 10.

Comme indiqué préalablement, à partir du moment où on a sélectionné les points « proches voisins », on peut facilement faire la moyenne des propriétés de ces proches voisins sélectionnés (étalons et/ou intergermes et/ou extragermes et/ou pôles) pour déterminer la propriété de l'échantillon inconnu (le produit cible). On reconstitue donc un spectre calculé du produit cible auquel correspond la caractéristique (propriété) ainsi calculée.

Toutefois, et ceci constitue un mode préféré d'exécution de la présente invention, la Demanderesse a découvert de manière inattendue une amélioration significative de la précision et robustesse de sa méthode lors de la détermination de la caractéristique recherchée (par exemple, une propriété) d'un produit cible lorsqu'on effectue une moyenne pondérée des propriétés de ces points « proches voisins » (qu'ils soient étalons et/ou intergermes et/ou extragermes et/ou pôles), la dite pondération étant une fonction inversement proportionnelle linéaire ou non-linéaire à la distance entre l'échantillon (« le produit cible ») et les points « proches voisins » sélectionnés ; cette pondération peut par exemple se représenter par la formule

$$\text{POND} \;=\; \frac{\frac{1}{di^{\alpha}}}{\sum_{1}^{n}\frac{1}{di^{\alpha}}}$$

Avec $\alpha$ étant un nombre positif de préférence compris entre 0.5 et 1.5,

di étant la distance entre le produit cible et le proche voisin i, et

n étant le nombre total de proches voisins.

On applique donc une pondération de ce type aux propriétés mesurées (et optionnellement calculées) des « proches voisins » pour obtenir la propriété du produit cible.

On reconstitue donc un spectre calculé du produit cible auquel correspond la caractéristique (propriété) ainsi calculée.

En d'autres termes, le calcul de la caractéristique Z du produit cible se fait grâce aux caractéristiques correspondantes Zi des points proches voisins, tout en donnant aux caractéristiques des dits points proches voisins un poids d'autant plus important dans le dit calcul qu'ils sont plus proches du produit cible.

Ainsi un mode d'exécution de la présente invention concerne également une méthode de caractérisation d'un produit cible comprenant les étapes suivantes :

1. Constitution d'une banque de données spectrales comprenant des échantillons, leurs spectres et leurs caractéristiques mesurées (« CAR », par exemple la propriété « P »),
2. Analyse spectrale du produit cible et comparaison du spectre obtenu (Spectre PC) avec les données spectrales de la banque de données,
3. Identification des points « proches voisins » du produit cible, et
4. Calcul par topologie de la caractéristique du produit cible (CARpc/top , par exemple la propriété Ppc/top) en fonction des caractéristiques correspondantes des points proches voisins,

caractérisé en ce que le calcul de l'étape 4 est basé sur une pondération liée à l'inverse de la distance entre le produit cible et les points proches voisins.

On peut utiliser la méthode de l'invention pour déterminer plus d'une propriété P à la fois, par exemple au moins 2, en particulier de 1 à 30 par exemple 2 à 10 propriétés à la fois. On pourra bien évidemment utiliser des nombres différents d'étalons choisis pour chaque propriété.

Selon un autre mode préféré d'exécution de la présente invention, la Demanderesse a découvert une méthode alternative particulièrement efficace.

Cette méthode consiste à combiner l'une des méthodes topologiques de caractérisation du produit cible susmentionnées avec n'importe quel modèle mathématique différent des méthodes topologiques (de préférence un modèle régressionnel) et permettant de caractériser le produit cible à partir des valeurs de grandeurs spectrales VGS (pour la même propriété).

Cette méthode implique donc la constitution préalable d'un modèle mathématique qui permet de calculer les propriétés des produits en fonction des valeurs de grandeur spectrale (VGS) de la banque de données, de préférence un modèle régressionnel (de caractérisation de produit à partir de la banque de données spectrales préétablie) ; cette banque de données spectrales peut être soit la banque de données A susmentionnée ou de préférence la banque de données A', E, EE ou EEI, ou une sélection des dites banques. De préférence, cette banque de données sera la même que celle qui a servi pour la méthode topologique.

Cette méthode alternative pour caractériser un produit cible comprend les étapes suivantes :

1. Constitution d'une banque de données spectrales comprenant des échantillons, leurs spectres et leurs caractéristiques mesurées (« CAR », par exemple la propriété « P »),
2. Analyse spectrale du produit cible et comparaison du spectre obtenu (Spectre PC) avec les données spectrales de la banque de données,
3. Identification des points « proches voisins » du produit cible,
4. Calcul par topologie
4.1. de la caractéristique du produit cible (CARpc/top , par exemple la propriété Ppc/top), et
4.2. de son spectre ainsi calculé (spectre PCcalc),
5. Etablissement à partir de la banque de données spectrales d'un modèle mathématique permettant de calculer la caractéristique d'un produit à partir de la banque de données spectrales (CAR/mod, par exemple propriété P/mod),
6. Calcul de la caractérisation du produit cible PC en suivant la formule suivante

$$CARpc = CARpc/top + [ \, CARpc/mod - CARpccalc/mod \, ]$$

avec

- CARpc étant la valeur calculée de la caractéristique du produit cible recherchée,
- CARpc/top étant la valeur calculée par topologie (points proches voisins) de la caractéristique du produit cible,
- CARpc/mod étant la valeur calculée par le modèle mathématique de la caractéristique du produit cible, et
- CARpccalc/mod étant la valeur calculée par le modèle mathématique de la caractéristique du produit cible calculé (au moyen des données spectrales obtenues au point 4.2).

La caractérisation d'un produit selon un mode d'exécution de la présente invention peut donc consister en une détermination et/ou une prédiction de toute caractéristique chimique, physique ou physico-chimique du dit produit et/ou l'identification d'un type et/ou famille de produits.

On peut par exemple déterminer la présence de composés chimiques individuels au sein d'une composition ainsi que leurs concentrations ; on peut également déterminer toute sorte de propriété dont certaines sont exemplifiées ci-après.

Ainsi on peut utiliser la méthode pour la détermination physico-chimique ou la prédiction en ce qui concerne au moins une charge d'alimentation ou un produit utilisé dans un processus industriel du raffinage du pétrole et/ou dans des opérations pétrochimiques ou obtenu à l'aide de ceux-ci. Le processus peut être une conversion d'hydrocarbure ou un processus de séparation, de préférence un processus de reformage ou de craquage catalytique ou d'hydrotraitement ou de distillation ou mélange. En particulier, on peut l'utiliser pour déterminer au moins une propriété d'une charge d'alimentation et/ou la prédiction et/ou la détermination d'au moins une propriété et/ou un rendement d'un produit provenant d'un certain nombre de processus différents tels que des processus pour séparer des produits pétroliers tels que la distillation atmosphérique, la distillation sous vide ou la séparation par distillation, sous une pression supérieure à la pression atmosphérique, ainsi que la conversion thermique ou catalytique, sans ou avec hydrogénation partielle ou totale, d'un produit pétrolier, telle que le craquage catalytique par exemple le craquage catalytique de fluide (FCC), l'hydrocraquage, le reformage, l'isomérisation, l'hydrogénation sélective, la viscoréduction ou l'alkylation. En particulier, la présente invention s'appliquera au mélange de constituants de produits cibles (par exemple des produits pétroliers cibles, par exemple des carburants) dans tout lieu approprié, par exemple une raffinerie, un dépôt pétrolier et/ou tout dispositif utilisant un produit cible constitué d'un mélange de constituants préparé en par batch et/ou de préférence en ligne.

L'utilisation de la méthode dans des opérations de mélange impliquant la production et/ou la détermination d'au moins une propriété d'un mélange d'hydrocarbures liquides (éventuellement avec d'autres additifs tels que des éthers alkyliques) est d'une valeur particulière, cette méthode comprenant ou non la détermination pour chaque constituant du mélange d'un indice de mélange pour la propriété envisagée. Dans cette méthode telle qu'appliquée au mélange, on peut obtenir simplement par calcul les indices de mélange et sans avoir besoin de préparer des mélanges physiques d'étalons autres que ceux contenus dans la base de données. On peut combiner linéairement (ou non linéairement) les indices de mélange dans les domaines de stabilité afin de déterminer à partir de la valeur de cette combinaison, une valeur pour au moins une propriété du mélange obtenu. On peut réaliser le mélange en mélangeant au moins deux composés parmi le butane, l'essence vapocraquée hydrogénée, l'isomérat, le reformat, le méthyl-ter-butyl-éther (MTBE) et/ou le méthyl-ter-amyl-éther (TAME) et/ou l'éthyl-ter-butyl-éther (ETBE), l'essence dérivée par FCC, l'éthanol et/ou les bioesters. On peut répéter ce processus en ajoutant numériquement les autres constituants séparément à la base d'hydrocarbure liquide pour déterminer une série d'indices de mélange et ensuite déterminer à partir de ces indices les propriétés du mélange multi-constituants.

Des exemples de propriétés que l'on peut déterminer et/ou prévoir sont les suivants: pour les carburants/essences d'automobiles, au moins l'un parmi l'indice d'octane de recherche (RON), l'indice d'octane de moteur (MON) et/ou leur moyenne arithmétique, avec ou sans additif et/ou la teneur en éther méthyl-t-butylique ou en éther methylisoamylique et/ou en benzène.

Pour les carburants/essences d'automobiles, au moins l'un parmi la tension de vapeur, la masse volumique, la volatilité, la courbe de distillation, par exemple le pourcentage distillé à 70°C et/ou à 100°C, la teneur en oxygène ou la teneur en benzène ou en soufre, la composition chimique et/ou la teneur en gomme par exemple exprimée en mg/100 ml (on détermine en particulier ces propriétés pour les utiliser dans les opérations de mélange).

Pour les carburants diesel ou gazole, au moins l'un parmi l'indice de cétane (par exemple, mesure au niveau du moteur), l'indice de cétane calculé, le point de trouble, le "point de décharge", l'aptitude au filtrage, la courbe de distillation, la masse volumique par exemple à 15°C, le point éclair, la viscosité par exemple à 40°C, la composition chimique, la sensibilité aux additifs et le pourcentage de soufre.

Pour les produits de distillation provenant du pétrole brut par exemple sous pression atmosphérique, au moins l'un parmi la masse volumique, le pourcentage de soufre, la viscosité à 100°C, la courbe de distillation, la teneur en paraffine, la teneur résiduelle en carbone ou la teneur en carbone de Conradson, la teneur en naphte, le point éclair de l'huile, le point de trouble pour le gazole, par exemple le gazole léger et/ou la viscosité à 100°C et/ou la teneur en soufre pour les résidus atmosphériques et le rendement pour au moins une des coupes, l'essence (Eb. 38 à 95°C), le benzène (Eb. 95 à 149°C), le naphta (Eb. 149 a 175 C), le kérosène (Eb. 175 à 232°C), le gazole léger (Eb. 232 à 342°C), le gazole lourd (Eb. 342 à 369°C) et celui du résidu atmosphérique supérieur à 369°C.

Pour au moins l'un parmi une charge d'alimentation ou un produit d'un processus de craquage catalytique par exemple un processus FCC, au moins l'un parmi la masse volumique, le pourcentage en soufre, le point d'aniline, l'indice de gazole, l'indice d'essence, la viscosité à 100°C, l'indice de réfraction à 20°C et/ou à 60°C, la masse moléculaire, la température de distillation par exemple la température de distillation à 50%, le pourcentage en carbone aromatique, la teneur totale en azote et les facteurs caractérisant l'aptitude de la charge d'alimentation au craquage par exemple le KUOP, le facteur de craquabilite, le facteur de cokabilite, et le rendement par exemple en gaz, en essence, en gazole ou en résidu. Ainsi on peut déterminer les rendements et/ou les propriétés des différents produits obtenus par la distillation de produits craques tels que RON et/ou MON, sans additif antidétonant pour la coupe d'essence et la viscosité à 100°C pour le résidu de distillation.

Pour au moins l'un parmi un produit ou une charge d'alimentation d'un processus de reformage catalytique, au moins l'un parmi la masse volumique, la température de distillation et/ou la composition chimique (exprimée en pourcentage) des hydrocarbures satures linéaires, des isoparaffines, des naphtènes, des substances aromatiques et des oléfines.

Pour au moins l'un parmi un produit ou une charge d'alimentation d'un processus d'hydrogénation d'essence, au moins l'un parmi la masse volumique, la température de distillation, RON et/ou MON, la tension de vapeur d'essence sans additif antidétonant ou plombée, la volatilité, la composition chimique (exprimée en pourcentage), en hydrocarbures saturés linéaires, en isoparaffines, en naphtènes, en substances aromatiques par exemple le benzène et les benzènes mono/di-substitues, en oléfines par exemple les oléfines cycliques et non cycliques, en dioléfines, et l'indice d'anhydride maléique.

Il doit être évident pour l'homme du métier que la présente invention permet des modes de réalisation sous de nombreuses autres formes spécifiques sans l'éloigner du domaine d'application de l'invention tel que revendiqué. Ainsi, les présents modes de réalisation doivent être considérés à titre d'illustration mais peuvent être modifiés dans le domaine défini par la portée des revendications jointes.

## Revendications

1. Procédé M de certification et de préparation d'un produit cible par mélange par batch et/ou en ligne de ses « n » constituants à partir de différents flux des dits constituants à des concentrations et/ou des débits contrôlés, ledit produit cible devant posséder en vue de sa commercialisation un ensemble de gammes de valeurs de caractéristiques physico-chimiques, dans lequel on alimente un mélangeur par batch et/ou en continu avec les dits constituants à des concentrations et/ou des débits contrôlés, et dans lequel :

   (1) au moins une donnée spectrale caractérisant le produit cible et qui définit son domaine spectral correspondant à l'ensemble de gammes de valeurs des caractéristiques physico-chimiques possédée par ledit produit cible en vue de sa commercialisation, est mesurée,
   (2) on dispose de données spectrales caractérisant chacune de manière individuelle au moins deux constituants du produit cible,
   (3) on utilise un programme informatique permettant de calculer les gammes de proportions respectives des dits constituants nécessaires à la reconstitution d'une donnée spectrale de mélange appartenant au domaine spectral de l'étape (1) à partir des données spectrales de l'étape (2), et
   (4) on utilise les gammes de proportions respectives des constituants de l'étape (3) pour contrôler les concentrations et/ou débits des constituants alimentant le mélangeur de manière à préparer le produit cible ayant une valeur de la dite donnée spectrale appartenant au domaine spectral de l'étape (1) et possédant par conséquence des valeurs des caractéristiques physico-chimiques dans l'ensemble de gammes de valeurs nécessaire, en vue de sa commercialisation et de la certification correspondante.

2. Procédé selon la revendication précédente **caractérisé en ce que** les données spectrales sont mesurées au moyen du même type d'analyse spectrale, de préférence au moyen du même type de spectromètre.

3. Procédé selon l'une quelconque des revendications précédentes **caractérisé en ce que** les données spectrales sont des spectres et/ou des banques de données spectrales.

4. Procédé selon l'une quelconque des revendications précédentes **caractérisé en ce que** les données spectrales proviennent d'analyse spectrale choisie parmi la RMN, le Raman, l'infrarouge (IR), le proche infrarouge (NIR) et/ou l'UV/Visible, de préférence d'une analyse spectrale topologique dans le proche infrarouge (« NIR »).

**5.** Procédé selon l'une quelconque des revendications précédentes **caractérisé en ce que** dans l'étape (2) le nombre de constituants « n » du produit cible est supérieur ou égal à 3.

**6.** Procédé selon l'une quelconque des revendications précédentes **caractérisé en ce qu'**on effectue une étape additionnelle consécutive à la deuxième étape (2) de la revendication 1, la dite étape additionnelle consistant en la constitution à partir des données spectrales caractérisant les constituants (de préférence à partir des banques de données spectrales caractérisant les constituants) de nouvelles données spectrales caractérisant les mélanges des dits constituants (de préférence des nouvelles banques de données spectrales caractérisant les mélanges des dits constituants), et à ensuite utiliser ces nouvelles données spectrales (de préférence ces nouvelles banques de données spectrales) dans la troisième (3) étape de la revendication 1.

**7.** Procédé selon l'une quelconque des revendications précédentes **caractérisé en ce que** le produit cible est un produit pétrolier, par exemple un carburant, par exemple un gazole et/ou une essence.

**8.** Procédé selon l'une quelconque des revendications précédentes **caractérisé en ce que** la préparation s'effectue dans une raffinerie, un complexe (pétro-)chimique, un dépôt pétrolier et/ou toute installation utilisant un produit constitué d'un mélange de constituants préparé par batch et/ou en ligne, en particulier dans un terminal ou toute installation de mélange post-raffinerie de carburants.

**9.** Utilisation d'un procédé selon l'une quelconque des revendications précédentes pour la préparation d'un produit cible consistant à combiner le dit procédé avec une ou plusieurs étapes additionnelles consistant à utiliser une ou plusieurs méthodes conventionnelles de mesure et/ou de vérification de(s) la propriété(s) Pz de manière à sélectionner, parmi les dites gammes de proportions respectives des constituants alimentant le mélangeur, la gamme de proportions à laquelle correspond un produit cible dont la(les) propriété(s) Pz satisfait à la contrainte dictée par la certification du dit produit cible, la dite propriété Pz étant de préférence la teneur en soufre du produit pétrolier dont la mesure - de préférence en ligne - est effectuée de préférence par détermination par spectrométrie de fluorescence X dispersive en longueur d'onde de la teneur en soufre dans les produits pétroliers.

**10.** Utilisation d'un procédé selon l'une quelconque des revendications 1 à 8 pour la préparation d'un produit cible consistant à privilégier [par exemple minimiser, maximiser et/ou moyenner] une (ou plusieurs) propriété(s) « Px », le procédé de certification et de préparation incluant une étape additionnelle consistant à sélectionner parmi les dites gammes de proportions respectives des constituants alimentant le mélangeur, la gamme de proportions à laquelle correspondra la valeur privilégiée de la (des) propriétés « Px », la(les) dite(s) propriétés(s) ayant été mesurées et/ou calculées en utilisant les données spectrales qui ont servi à définir les gammes de proportions respectives des constituants nécessaires à la préparation d'un produit cible.

**11.** Utilisation d'un procédé selon l'une quelconque des revendications 1 à 8 pour la préparation d'un produit cible comprenant un constituant « X », consistant à privilégier l'utilisation dudit constituant « X » parmi les « n » constituants du mélange produit cible, le procédé de préparation incluant une étape additionnelle consistant à sélectionner parmi les gammes de proportions respectives des constituants alimentant le mélangeur de l'étape (3) de la revendication 1, la gamme de proportion ayant la concentration en constituant « X » la plus élevée.

**12.** Utilisation d'un procédé selon l'une quelconque des revendications 1 à 8

    a. pour valider le remplacement d'un constituant « X » par un constituant « X' », et/ou
    b. prédire le taux d'utilisation potentiel du constituant « X' »

lors de la préparation d'un produit cible comprenant initialement un constituant « X », le procédé de préparation incluant

    a. une étape d'analyse spectrale du constituant « X' » pour en déterminer une donnée spectrale caractérisante, par exemple un spectre et/ou une banque de données spectrales caractérisante,
    b. une étape consistant en une étape de remplacement de la donnée spectrale caractérisante du constituant « X » par la donnée spectrale caractérisante du constituant « X' » dans le procédé,
    c. une étape de validation du remplacement d'un constituant « X » par un constituant « X' » et/ou de prédiction le taux d'utilisation potentiel du constituant « X' » grâce à la troisième étape (3) de la revendication 1 qui permet de vérifier qu'il existe au moins une gamme de proportions respectives des nouveaux constituants nécessaires à la reconstitution d'une donnée spectrale du mélange dans la gamme de valeurs de l'étape (1) appartenant au domaine spectral du produit cible.

**Patentansprüche**

1. Verfahren M zur Zertifizierung und Herstellung eines Zielprodukts durch chargenweises und/oder kontinuierliches Mischen seiner "n" Bestandteile ausgehend von verschiedenen Strömen der Bestandteile mit kontrollierten Konzentrationen und/oder Volumenströmen, wobei das Zielprodukt im Hinblick auf seine Vermarktung eine Gesamtheit von Wertebereichen chemisch-physikalischer Eigenschaften besitzen muss, bei dem einem Mischer chargenweise und/oder ohne Unterbrechung die Bestandteile mit kontrollierten Konzentrationen und/oder Volumenströmen zugeführt werden und bei dem:

   (1) mindestens ein Spektraldatenwert, der das Zielprodukt charakterisiert und der seinen Spektralbereich definiert, der einer Gesamtheit von Wertebereichen der chemisch-physikalischen Eigenschaften entspricht, die das Zielprodukt im Hinblick auf seine Vermarktung besitzt, gemessen wird,
   (2) Spektraldaten erhalten werden, die jeweils mindestens zwei Bestandteile des Zielprodukts individuell charakterisieren,
   (3) ein Computerprogramm verwendet wird, das es ermöglicht, die Bereiche jeweiliger Anteile der Bestandteile, die zur Nachbildung eines Mischungsspektraldatenwerts erforderlich sind, der dem Spektralbereich des Schritts (1) angehört, ausgehend von den Spektraldaten des Schritts (2) zu berechnen, und
   (4) die Bereiche jeweiliger Anteile der Bestandteile des Schritts (3) verwendet werden, um die Konzentrationen und/oder Volumenströme der Bestandteile, die dem Mischer zugeführt werden, derart zu kontrollieren, dass ein Zielprodukt hergestellt wird, das einen Wert für den Spektraldatenwert besitzt, der dem Spektralbereich des Schritts (1) angehört, und infolgedessen Werte der chemisch-physikalischen Eigenschaften in der Gesamtheit von Wertebereichen besitzt, die im Hinblick auf seine Vermarktung und die entsprechende Zertifizierung erforderlich ist.

2. Verfahren nach dem vorhergehenden Anspruch, **dadurch gekennzeichnet, dass** die Spektraldaten mit dem gleichen Spektralanalysetyp gemessen werden, bevorzugt mit dem gleichen Spektrometertyp.

3. Verfahren nach einem der vorhergehenden Ansprüche, **dadurch gekennzeichnet, dass** die Spektraldaten Spektren und/oder Spektraldatenbanken sind.

4. Verfahren nach einem der vorhergehenden Ansprüche, **dadurch gekennzeichnet, dass** die Spektraldaten aus einer Spektralanalyse stammen, die unter NMR, Raman, Infrarot (IR), nahem Infrarot (NIR) und/oder UV/VIS gewählt ist, bevorzugt aus einer topologischen Spektralanalyse im nahem Infrarot ("NIR").

5. Verfahren nach einem der vorhergehenden Ansprüche, **dadurch gekennzeichnet, dass** die Anzahl von Bestandteilen "n" des Zielprodukts im Schritt (2) größer als oder gleich 3 ist.

6. Verfahren nach einem der vorhergehenden Ansprüche, **dadurch gekennzeichnet, dass** nach dem zweiten Schritt (2) des Anspruchs 1 ein zusätzlicher Schritt durchgeführt wird, wobei der zusätzliche Schritt in der Bildung, ausgehend von den Spektraldaten, welche die Bestandteile charakterisieren (bevorzugt ausgehend von den Spektraldatenbanken, welche die Bestandteile charakterisieren), von neuen Spektraldaten besteht, welche die Mischungen der Bestandteile charakterisieren (bevorzugt von neuen Spektraldatenbanken, welche die Mischungen der Bestandteile charakterisieren), und anschließend diese neuen Spektraldaten (bevorzugt diese neuen Spektraldatenbanken) im dritten (3) Schritt des Anspruchs 1 zu verwenden.

7. Verfahren nach einem der vorhergehenden Ansprüche, **dadurch gekennzeichnet, dass** das Zielprodukt ein Mineralölprodukt ist, beispielsweise ein Kraftstoff, beispielsweise ein Diesel und/oder ein Benzin.

8. Verfahren nach einem der vorhergehenden Ansprüche, **dadurch gekennzeichnet, dass** die Herstellung in einer Raffinerie, einem (petro-)chemischen Komplex, einem Mineralöllager und/oder einer Anlage durchgeführt wird, die ein Produkt verwendet, das aus einer Mischung von Bestandteilen besteht, die chargenweise und/oder kontinuierlich hergestellt wird, insbesondere in einer der Raffinerie nachgeschalteten Kraftstoffmischeinrichtung oder -anlage.

9. Verwendung eines Verfahrens nach einem der vorhergehenden Ansprüche zur Herstellung eines Zielprodukts, darin bestehend, das Verfahren mit einem oder mehreren zusätzlichen Schritten zu kombinieren, die darin bestehen, eine oder mehrere konventionelle Methoden zum Messen und/oder Verifizieren der Eigenschaft(en) Pz derart zu verwenden, dass unter den Bereichen jeweiliger Anteile der Bestandteile, die dem Mischer zugeführt werden, der Anteilsbereich ausgewählt wird, dem ein Zielprodukt entspricht, dessen Eigenschaft(en) Pz die durch die Zertifizierung des Zielprodukts vorgegebene Beschränkung erfüllt(erfüllen), wobei die Eigenschaft Pz bevorzugt der Schwefelgehalt des Mineralölprodukts ist, dessen Messung - bevorzugt kontinuierlich - bevorzugt durch Bestimmung durch wellenlängendispersive Röntgenfluoreszenzspektrometrie des Schwefelgehalts in den Mineralölprodukten durchgeführt wird.

10. Verwendung eines Verfahrens nach einem der Ansprüche 1 bis 8 zur Herstellung eines Zielprodukts, darin bestehend, eine (oder mehrere) Eigenschaft(en) "Px" zu bevorzugen [beispielsweise zu minimieren, zu maximieren und/oder zu mitteln], wobei das Zertifizierungs- und Herstellungsverfahren einen zusätzlichen Schritt beinhaltet, der darin besteht, unter den Bereichen jeweiliger Anteile der Bestandteile, die dem Mischer zugeführt werden, den Anteilsbereich auszuwählen, dem der bevorzugte Wert der Eigenschaft(en) "Px" entspricht, wobei die Eigenschaft(en) unter Verwendung der Spektraldaten gemessen und/oder berechnet worden sind, die dazu gedient haben, die Bereiche jeweiliger Anteile der Bestandteile zu definieren, die zur Herstellung eines Zielprodukts erforderlich sind.

11. Verwendung eines Verfahrens nach einem der Ansprüche 1 bis 8 zur Herstellung eines einen Bestandteil "X" umfassenden Zielprodukts, darin bestehend, die Verwendung des Bestandteils "X" unter den "n" Bestandteilen der Zielproduktmischung zu bevorzugen, wobei das Herstellungsverfahren einen zusätzlichen Schritt beinhaltet, der darin besteht, unter den Bereichen jeweiliger Anteile der dem Mischer zugeführten Bestandteile des Schritts (3) des Anspruchs 1 den Anteilsbereich auszuwählen, der die höchste Konzentration des Bestandteils "X" hat.

12. Verwendung eines Verfahren nach einem der Ansprüche 1 bis 8

 a. zum Validieren der Ersetzung eines Bestandteils "X" durch einen Bestandteil "X'" und/oder
 b. zum Vorhersagen des potenziellen Verwendungsgrads des Bestandteils "X'" bei der Herstellung eines Zielprodukts, das anfänglich einen Bestandteil "X" umfasst, wobei das Herstellungsverfahren beinhaltet
 a. einen Schritt der Spektralanalyse des Bestandteils "X'", um einen charakterisierenden Spektraldatenwert davon zu bestimmen, beispielsweise ein Spektrum und/oder eine charakterisierende Spektraldatenbank,
 b. einen Schritt, der in einem Schritt des Ersetzens des charakterisierenden Spektraldatenwerts des Bestandteils "X" durch den charakterisierenden Spektraldatenwert des Bestandteils "X'" in dem Verfahren besteht,
 c. einen Schritt des Validierens der Ersetzung eines Bestandteils "X" durch einen Bestandteil "X'" und/oder des Vorhersagens des potenziellen Verwendungsgrads des Bestandteils "X'" dank des dritten Schritts (3) des Anspruchs 1, der es ermöglicht zu verifizieren, dass es mindestens einen Bereich jeweiliger Anteile der neuen Bestandteile gibt, die zur Nachbildung eines Spektraldatenwerts der Mischung in dem Wertebereich des Schritts (1) erforderlich sind, der dem Spektralbereich des Zielprodukts angehört.

## Claims

1. Method M for certification and preparation of a target product for batch and/or in-line mixing of its "n" components from different flows from said components at controlled concentrations and/or flow rates, said target product having to be marketed with a set of ranges of physico-chemical characteristic values, in which a batch and/or continuous mixer is fed said components at controlled concentrations and/or flow rates, wherein:

 (1) at least one spectral datum characterizing the target product and which defines its spectral range corresponding to the set of ranges of physico-chemical characteristic values of the said target product having to be marketed, is measured,
 (2) spectral data are available, each individually characterizing at least two components of the target product,
 (3) a computer program is used which makes it possible to calculate the ranges of respective proportions of said components necessary for reconstituting a spectral datum of the mixture belonging to the spectral range of Step (1) from the spectral data of Step (2), and
 (4) the ranges of respective proportions of the constituents of Step (3) are used to control the concentrations and/or flow rates of the components fed into the mixer so as to prepare the target product whose value of said spectral datum belonging to the spectral range of Step (1) and consequently having physico-chemical characteristic values in the required set of ranges for its marketing and corresponding certification.

2. Method according to the foregoing Claim **characterized in that** the spectral data are measured using the same type of spectral analysis, preferably using the same type of spectrometer.

3. Method according to any of the preceding Claims **characterized in that** the spectral data are spectra and/or spectral databases.

4. Method according to any of the preceding Claims **characterized in that** the spectral data are obtained from spectral analysis chosen from NMR, Raman, infrared (IR), near-infrared (NIR) and/or UV/Visible, preferably near-infrared topological spectral analysis ("NIR").

5. Method according to any of the preceding Claims **characterized in that** in Step (2) the number of "n" components of the target product is greater than or equal to three.

6. Method according to any of the preceding Claims **characterized in that** it performs an additional step following the second step (2) of Claim 1, said additional step consisting of the constitution from the spectral data characterizing the components (preferably from the spectral databases characterizing the components) of new spectral data characterizing the mixtures of said components (preferably new spectral databases characterizing the mixtures of said components), and then using these new spectral data (preferably these new spectral databases) in the third step (3) of Claim 1.

7. Method according to any of the preceding Claims **characterized in that** the target product is a petroleum product, for example a fuel, for example a gas oil and/or a gasoline.

8. Method according to any of the preceding Claims **characterized in that** the preparation is carried out in a refinery, a (petro-) chemical complex, a petroleum depot and/or any plant using a batch and/or in-line mixture of components, in particular in a terminal or any post-refinery fuel mixing facility.

9. Use of a method according to any of the preceding Claims for preparing a target product comprising the combination of said method with one or more additional steps of using one or more conventional methods to measure and/or verify the property(ies) Pz so as to select,
from among said ranges of respective proportions of the components feeding the mixer, the range of proportions to which a target product whose Pz property(ies) satisfies the constraint dictated by the certification of said target product corresponds, said Pz property preferably being the sulfur content of the petroleum product whose measurement - preferably in-line - is preferably carried out by wavelength-dispersive x-ray fluorescence spectrometric determination of the sulfur content in the petroleum products.

10. Use of a method according to any of Claims 1 to 8 for the preparation of a target product which consists in focusing [for example, minimizing, maximizing and/or averaging] on one or more "Px" properties, the method of certification and preparation will include an additional step which will consist in selecting from said respective ranges of component proportions being fed into the mixer, the range of proportions at which the preferred value of the "Px" properties will correspond, the said property(ies) having been measured and/or calculated using the spectral data used to define the range of properties of the respective proportions of the components required for the preparation of a target product.

11. Use of a method according to any of Claims 1 to 8 for the preparation of a target product comprising a component "X", consisting in favoring the use of said component "X" from among the "n" components of the target product mixture, the preparation method including an additional step consisting in selecting from the ranges of respective proportions of the components being fed into the mixer of step (3) of Claim 1, the proportion range having the highest concentration of "X" component.

12. Use of a method according to any of Claims 1 to 8

a. to validate the replacement of component "X" by component "X'", and/or
b. predicting the potential use rate of component "X'"

during preparation of a target product initially comprising component "X", the preparation process including

a. a spectral analysis step of component "X'" to determine a characterizing spectral datum, for example a spectrum and/or a characterizing spectral databank,
b. a step consisting of a step to replace the spectral datum characterizing component "X" with the spectral datum characterizing component "X'" in the Process,

c. a validation step to replace an "X" component with an "X'" component and/or to predict the potential rate of use of the component "X'" for the third step (3) of Claim 1 that makes it possible to verify that at least one range exists of the respective proportions of the new components necessary to the reconstitution of a spectral datum of the mixture in the range of values of Step (1) belonging to the spectral domain of the target product.

FIGURE 1

| Absorbances | Nombre d'onde (cm-1) | 4776 | 4772 | 4768 | 4764 | 4760 | 4756 | 4752 | 4748 | 4744 | 4740 | 4736 | 4732 | 4728 |
|---|---|---|---|---|---|---|---|---|---|---|---|---|---|---|
| Etalon | | | | | | | | | | | | | | |
| A0000001 | | 0.00000131 | 0.00000234 | 0.00000471 | 0.00000778 | 0.00001291 | 0.00001881 | 0.00002420 | 0.00003164 | 0.00004422 | 0.00006178 | 0.00008012 | 0.00009882 | 0.00011603 |
| A0000002 | | 0.00000154 | 0.00000300 | 0.00000484 | 0.00000808 | 0.00001365 | 0.00001962 | 0.00002497 | 0.00003183 | 0.00004456 | 0.00006093 | 0.00007915 | 0.00009801 | 0.00011606 |
| A0000003 | | 0.00000131 | 0.00000284 | 0.00000512 | 0.00000878 | 0.00001523 | 0.00002234 | 0.00002966 | 0.00003987 | 0.00005529 | 0.00007543 | 0.00009683 | 0.00011817 | 0.00013911 |
| A0000004 | | 0.00000028 | 0.00000173 | 0.00000375 | 0.00000746 | 0.00001259 | 0.00001808 | 0.00002468 | 0.00003287 | 0.00004706 | 0.00006552 | 0.00008471 | 0.00010404 | 0.00012267 |
| A0000005 | | 0.00000152 | 0.00000346 | 0.00000608 | 0.00001005 | 0.00001590 | 0.00002388 | 0.00003277 | 0.00004342 | 0.00005927 | 0.00008077 | 0.00010398 | 0.00012785 | 0.00014973 |
| A0000006 | | 0.00000025 | 0.00000137 | 0.00000276 | 0.00000549 | 0.00000920 | 0.00001410 | 0.00001884 | 0.00002541 | 0.00003622 | 0.00005183 | 0.00006968 | 0.00008685 | 0.00010424 |
| A0000007 | | 0.00000115 | 0.00000192 | 0.00000381 | 0.00000714 | 0.00001208 | 0.00001862 | 0.00002413 | 0.00003267 | 0.00004633 | 0.00006518 | 0.00008486 | 0.00010414 | 0.00012311 |
| A0000008 | | 0.00000145 | 0.00000286 | 0.00000597 | 0.00001025 | 0.00001641 | 0.00002401 | 0.00003255 | 0.00004393 | 0.00006125 | 0.00008424 | 0.00010897 | 0.00013581 | 0.00016053 |
| A0000009 | | 0.00000196 | 0.00000443 | 0.00000613 | 0.00000825 | 0.00001145 | 0.00001403 | 0.00001617 | 0.00001990 | 0.00002562 | 0.00003560 | 0.00004941 | 0.00006621 | 0.00008322 |

:

:

| Absorbances | Nombre d'onde (cm-1) | 4352 | 4348 | 4344 | 4340 | 4336 | 4332 | 4328 | 4324 | 4320 | 4316 | 4312 | 4308 | 4304 |
|---|---|---|---|---|---|---|---|---|---|---|---|---|---|---|
| Etalon | | | | | | | | | | | | | | |
| A0000001 | | 0.00966521 | 0.01052209 | 0.01158039 | 0.01273609 | 0.01366893 | 0.01401955 | 0.01384877 | 0.01353086 | 0.01333914 | 0.01318977 | 0.01295103 | 0.01250628 | 0.01190033 |
| A0000002 | | 0.00962570 | 0.01045172 | 0.01147713 | 0.01259841 | 0.01351549 | 0.01386926 | 0.01370155 | 0.01337238 | 0.01316494 | 0.01301322 | 0.01279028 | 0.01237890 | 0.01181515 |
| A0000003 | | 0.00971714 | 0.01051752 | 0.01151046 | 0.01260728 | 0.01350349 | 0.01385874 | 0.01371417 | 0.01342094 | 0.01323014 | 0.01306308 | 0.01280913 | 0.01238076 | 0.01181617 |
| A0000004 | | 0.00970203 | 0.01053299 | 0.01156151 | 0.01269044 | 0.01360349 | 0.01396508 | 0.01381938 | 0.01353130 | 0.01333327 | 0.01313686 | 0.01284140 | 0.01236973 | 0.01178167 |
| A0000005 | | 0.00983396 | 0.01059963 | 0.01155613 | 0.01260533 | 0.01346771 | 0.01381668 | 0.01367895 | 0.01339166 | 0.01320615 | 0.01304760 | 0.01281606 | 0.01240677 | 0.01184798 |
| A0000006 | | 0.00975312 | 0.01059458 | 0.01165282 | 0.01283536 | 0.01379368 | 0.01414591 | 0.01392945 | 0.01352795 | 0.01322576 | 0.01296382 | 0.01265066 | 0.01220577 | 0.01166779 |
| A0000007 | | 0.00971298 | 0.01053791 | 0.01157010 | 0.01271768 | 0.01364754 | 0.01400085 | 0.01381945 | 0.01347245 | 0.01322868 | 0.01300856 | 0.01271797 | 0.01227517 | 0.01172447 |
| A0000008 | | 0.00973713 | 0.01055416 | 0.01158105 | 0.01271146 | 0.01362481 | 0.01395885 | 0.01376642 | 0.01341727 | 0.01316979 | 0.01298435 | 0.01274264 | 0.01233134 | 0.01177974 |
| A0000009 | | 0.00973863 | 0.01054689 | 0.01155098 | 0.01265425 | 0.01355805 | 0.01391147 | 0.01373056 | 0.01336471 | 0.01310149 | 0.01290984 | 0.01267387 | 0.01227335 | 0.01172989 |

BANQUE DE DONNEES SPECTRALES A

FIGURE 2

| Longueur d'onde VGS | W1 | W2 | W3 | W4 | W5 | W6 | W7 | W8 | W9 |
|---|---|---|---|---|---|---|---|---|---|
| | 1.52455E-07 | 5.98E-06 | 5.43E-06 | 9.39E-06 | 1.38E-05 | 2.07E-05 | 2.62E-05 | 3.04E-05 | 4.14E-05 |
| | 1.12042E-07 | 6.81E-06 | 5.33E-06 | 9.54E-06 | 1.46E-05 | 2.15E-05 | 2.69E-05 | 3.11E-05 | 4.18E-05 |
| | 1.55755E-06 | 4.53E-06 | 6.07E-06 | 9.07E-06 | 1.45E-05 | 2.07E-05 | 2.6E-05 | 3.13E-05 | 4.12E-05 |
| | 4.15098E-08 | 6.65E-06 | 5.33E-06 | 9.63E-06 | 1.45E-05 | 2.09E-05 | 2.62E-05 | 3.12E-05 | 4.14E-05 |
| | 1.28445E-07 | 6.52E-06 | 5.19E-06 | 9.68E-06 | 1.46E-05 | 2.21E-05 | 2.7E-05 | 3.08E-05 | 4.2E-05 |
| | 1.52686E-06 | 4.24E-06 | 6.16E-06 | 8.66E-06 | 1.36E-05 | 1.97E-05 | 2.57E-05 | 3.07E-05 | 4.05E-05 |
| | 1.45709E-06 | 4.02E-06 | 6.06E-06 | 9.18E-06 | 1.47E-05 | 2.04E-05 | 2.63E-05 | 3.14E-05 | 4.1E-05 |
| | 1.8449E-06 | 4.52E-06 | 5.79E-06 | 8.99E-06 | 1.43E-05 | 2.02E-05 | 2.62E-05 | 3.16E-05 | 4.17E-05 |
| | 1.73044E-06 | 4.4E-06 | 6.12E-06 | 8.94E-06 | 1.42E-05 | 2.04E-05 | 2.63E-05 | 3.11E-05 | 4.07E-05 |
| | 1.11909E-06 | 3.85E-06 | 6.37E-06 | 9.31E-06 | 1.43E-05 | 2.08E-05 | 2.59E-05 | 3.07E-05 | 4.07E-05 |
| VGSm | 9.67038E-07 | 5.15E-06 | 5.79E-06 | 9.24E-06 | 1.43E-05 | 2.07E-05 | 2.63E-05 | 3.11E-05 | 4.12E-05 |
| $\sigma$ | 7.6254E-07 | 1.19E-06 | 4.27E-07 | 3.31E-07 | 3.79E-07 | 6.75E-07 | 4.21E-07 | 3.79E-07 | 4.99E-07 |
| $\frac{m}{\sigma} * 100$ | 78.85 | 23.09 | 7.38 | 3.58 | 2.65 | 3.26 | 1.60 | 1.22 | 1.21 |

BANQUE DE DONNEES SPECTRALES B

FIGURE 3

| Absorbances | Nombre d'onde (cm-1) | 4776 | 4772 | 4768 | 4764 | 4760 | 4756 | 4752 | 4748 | 4744 | 4740 | 4736 | 4732 | 4728 |
|---|---|---|---|---|---|---|---|---|---|---|---|---|---|---|
| Etalon | | | | | | | | | | | | | | |
| A0000001 | | 0.00000131 | 0.00000234 | 0.00000471 | 0.00000778 | 0.00001291 | 0.00001881 | 0.00002420 | 0.00003164 | 0.00004422 | 0.00006178 | 0.00008012 | 0.00009882 | 0.00011603 |
| A0000002 | | 0.00000154 | 0.00000300 | 0.00000484 | 0.00000808 | 0.00001365 | 0.00001962 | 0.00002497 | 0.00003183 | 0.00004456 | 0.00006093 | 0.00007915 | 0.00009801 | 0.00011606 |
| A0000003 | | 0.00000131 | 0.00000284 | 0.00000512 | 0.00000878 | 0.00001523 | 0.00002234 | 0.00002966 | 0.00003987 | 0.00005529 | 0.00007543 | 0.00009683 | 0.00011817 | 0.00013911 |
| A0000004 | | 0.00000028 | 0.00000173 | 0.00000375 | 0.00000746 | 0.00001259 | 0.00001808 | 0.00002468 | 0.00003287 | 0.00004706 | 0.00006552 | 0.00008471 | 0.00010404 | 0.00012267 |
| A0000005 | | 0.00000152 | 0.00000346 | 0.00000608 | 0.00001005 | 0.00001590 | 0.00002388 | 0.00003277 | 0.00004342 | 0.00005927 | 0.00008077 | 0.00010398 | 0.00012785 | 0.00014973 |
| A0000006 | | 0.00000025 | 0.00000137 | 0.00000276 | 0.00000549 | 0.00000920 | 0.00001410 | 0.00001884 | 0.00002541 | 0.00003622 | 0.00005183 | 0.00006968 | 0.00008685 | 0.00010424 |
| A0000007 | | 0.00000115 | 0.00000192 | 0.00000381 | 0.00000714 | 0.00001208 | 0.00001862 | 0.00002413 | 0.00003267 | 0.00004633 | 0.00006518 | 0.00008486 | 0.00010414 | 0.00012311 |
| A0000008 | | 0.00000145 | 0.00000286 | 0.00000597 | 0.00001025 | 0.00001641 | 0.00002401 | 0.00003255 | 0.00004393 | 0.00006125 | 0.00008424 | 0.00010897 | 0.00013581 | 0.00016053 |
| A0000009 | | 0.00000196 | 0.00000443 | 0.00000613 | 0.00000825 | 0.00001145 | 0.00001403 | 0.00001617 | 0.00001990 | 0.00002562 | 0.00003560 | 0.00004941 | 0.00006621 | 0.00008322 |

BANQUE DE DONNEES SPECTRALES AMELIOREE A'

FIGURE 4

| Absorbance Nom | %Poids | Germe 1 | Germe 2 | Germe 3 | 4764 | 4760 | 4756 | 4752 | 4748 | 4744 | 4740 | 4736 |
|---|---|---|---|---|---|---|---|---|---|---|---|---|
| A0000001 | | | | | 7.78E-06 | 1.29E-05 | 1.88E-05 | 2.42E-05 | 3.16E-05 | 4.42E-05 | 6.18E-05 | 8.01E-05 |
| A0000002 | | | | | 8.08E-06 | 1.37E-05 | 1.96E-05 | 2.5E-05 | 3.18E-05 | 4.46E-05 | 6.09E-05 | 7.91E-05 |
| A0000003 | | | | | 8.78E-06 | 1.52E-05 | 2.23E-05 | 2.97E-05 | 3.99E-05 | 5.53E-05 | 7.54E-05 | 9.68E-05 |
| A0000004 | | | | | 7.46E-06 | 1.26E-05 | 1.81E-05 | 2.47E-05 | 3.29E-05 | 4.71E-05 | 6.55E-05 | 8.47E-05 |
| A0000005 | | | | | 1.01E-05 | 1.59E-05 | 2.39E-05 | 3.28E-05 | 4.34E-05 | 5.93E-05 | 8.08E-05 | 0.000104 |
| A0000006 | | | | | 5.49E-06 | 9.2E-06 | 1.41E-05 | 1.88E-05 | 2.54E-05 | 3.62E-05 | 5.18E-05 | 6.97E-05 |
| A0000007 | | | | | 7.14E-06 | 1.21E-05 | 1.86E-05 | 2.41E-05 | 3.27E-05 | 4.63E-05 | 6.52E-05 | 8.49E-05 |
| A0000008 | | | | | 1.03E-05 | 1.64E-05 | 2.4E-05 | 3.26E-05 | 4.39E-05 | 6.13E-05 | 8.42E-05 | 0.000109 |
| A0000009 | | | | | 8.25E-06 | 1.15E-05 | 1.4E-05 | 1.62E-05 | 1.99E-05 | 2.56E-05 | 3.56E-05 | 4.94E-05 |
| I2G022 | 0.564<br>0.436 | A0000003 | A0000006 | | 7.35E-06 | 1.26E-05 | 1.88E-05 | 2.49E-05 | 3.36E-05 | 4.7E-05 | 6.51E-05 | 8.5E-05 |
| I2G011 | 0.654<br>0.346 | A0000009 | A0000001 | | 8.09E-06 | 1.2E-05 | 1.57E-05 | 1.9E-05 | 2.4E-05 | 3.21E-05 | 4.47E-05 | 6E-05 |
| I2G036 | 0.44<br>0.56 | A0000008 | A0000004 | | 8.69E-06 | 1.43E-05 | 2.07E-05 | 2.81E-05 | 3.77E-05 | 5.33E-05 | 7.38E-05 | 9.54E-05 |
| I3G038 | 0.747<br>0.258<br>-0.005 | A0000008 | A0000002 | A0000004 | 9.71E-06 | 1.57E-05 | 2.29E-05 | 3.06E-05 | 4.09E-05 | 5.7E-05 | 7.83E-05 | 0.000101 |
| I3G025 | 0.5825<br>0.3020<br>0.1155 | A0000008 | A0000005 | A0000003 | 1E-05 | 1.61E-05 | 2.38E-05 | 3.23E-05 | 4.33E-05 | 6E-05 | 8.22E-05 | 0.000106 |
| I3G019 | 0.094<br>-0.00047<br>0.00647 | A0000004 | A0000005 | A0000007 | 7.43E-06 | 1.25E-05 | 1.81E-05 | 2.46E-05 | 3.28E-05 | 4.7E-05 | 6.54E-05 | 8.46E-05 |

BANQUE DE DONNEES SPECTRALES ELARGIE E

FIGURE 5

EP 3 155 402 B1

| Absorbance Nom | Pole | Germe | %poids | 4764 | 4760 | 4756 | 4752 | 4748 | 4744 | 4740 | 4736 | 4732 |
|---|---|---|---|---|---|---|---|---|---|---|---|---|
| A0000001 | | | | 7.78E-06 | 1.29E-05 | 1.88E-05 | 2.42E-05 | 3.16E-05 | 4.42E-05 | 6.18E-05 | 8.01E-05 | 9.88E-05 |
| A0000002 | | | | 8.08E-06 | 1.37E-05 | 1.96E-05 | 2.5E-05 | 3.18E-05 | 4.46E-05 | 6.09E-05 | 7.91E-05 | 9.8E-05 |
| A0000003 | | | | 8.78E-06 | 1.52E-05 | 2.23E-05 | 2.97E-05 | 3.99E-05 | 5.53E-05 | 7.54E-05 | 9.68E-05 | 0.000118 |
| A0000004 | | | | 7.46E-06 | 1.26E-05 | 1.81E-05 | 2.47E-05 | 3.29E-05 | 4.71E-05 | 6.55E-05 | 8.47E-05 | 0.000104 |
| A0000005 | | | | 1.01E-05 | 1.59E-05 | 2.39E-05 | 3.28E-05 | 4.34E-05 | 5.93E-05 | 8.08E-05 | 0.000104 | 0.000128 |
| A0000006 | | | | 5.49E-06 | 9.2E-06 | 1.41E-05 | 1.88E-05 | 2.54E-05 | 3.62E-05 | 5.18E-05 | 6.97E-05 | 8.68E-05 |
| A0000007 | | | | 7.14E-06 | 1.21E-05 | 1.86E-05 | 2.41E-05 | 3.27E-05 | 4.63E-05 | 6.52E-05 | 8.49E-05 | 0.000104 |
| A0000008 | | | | 1.03E-05 | 1.64E-05 | 2.4E-05 | 3.26E-05 | 4.39E-05 | 6.13E-05 | 8.42E-05 | 0.000109 | 0.000136 |
| A0000009 | | | | 8.25E-06 | 1.15E-05 | 1.4E-05 | 1.62E-05 | 1.99E-05 | 2.56E-05 | 3.56E-05 | 4.94E-05 | 6.62E-05 |
| MEG001 | PAL054 | A0000009 | 0.15 | 4.45E-06 | 7.13E-06 | 9.35E-06 | 1.12E-05 | 1.44E-05 | 1.95E-05 | 2.84E-05 | 4.08E-05 | 5.62E-05 |
| MEG002 | PAL014 | A0000005 | -0.05 | 1.15E-05 | 1.78E-05 | 2.63E-05 | 3.57E-05 | 4.69E-05 | 6.36E-05 | 8.61E-05 | 0.00011 | 0.000135 |
| MEG003 | PAL035 | A0000008 | 0.08 | 8.11E-06 | 1.37E-05 | 2.06E-05 | 2.84E-05 | 3.88E-05 | 5.48E-05 | 7.6E-05 | 9.89E-05 | 0.000124 |
| MEG004 | PRF006 | A0000002 | 0.021655 | 8.65E-06 | 1.43E-05 | 2.03E-05 | 2.57E-05 | 3.26E-05 | 4.53E-05 | 6.16E-05 | 7.98E-05 | 9.88E-05 |
| MEG005 | PRF004 | A0000007 | -0.07268 | 4.86E-06 | 9.48E-06 | 1.56E-05 | 2.07E-05 | 2.94E-05 | 4.35E-05 | 6.29E-05 | 8.26E-05 | 0.000102 |
| MEG006 | PRF074 | A0000003 | 0.028752 | 9.54E-06 | 1.61E-05 | 2.33E-05 | 3.07E-05 | 4.07E-05 | 5.59E-05 | 7.58E-05 | 9.72E-05 | 0.000119 |

BANQUE DE DONNEES SPECTRALES ELARGIE EE

FIGURE 6

| Absorbance Nom | Germe1 | Germe2 | Germe3 | pole | %poids | 4764 | 4760 | 4756 | 4752 | 4748 | 4744 | 4740 |
|---|---|---|---|---|---|---|---|---|---|---|---|---|
| A0000001 | | | | | | 7.78E-06 | 1.29E-05 | 1.88E-05 | 2.42E-05 | 3.16E-05 | 4.42E-05 | 6.18E-05 |
| A0000002 | | | | | | 8.08E-06 | 1.37E-05 | 1.96E-05 | 2.5E-05 | 3.18E-05 | 4.46E-05 | 6.09E-05 |
| A0000003 | | | | | | 8.78E-06 | 1.52E-05 | 2.23E-05 | 2.97E-05 | 3.99E-05 | 5.53E-05 | 7.54E-05 |
| A0000004 | | | | | | 7.46E-06 | 1.26E-05 | 1.81E-05 | 2.47E-05 | 3.29E-05 | 4.71E-05 | 6.55E-05 |
| A0000005 | | | | | | 1.01E-05 | 1.59E-05 | 2.39E-05 | 3.28E-05 | 4.34E-05 | 5.93E-05 | 8.08E-05 |
| A0000006 | | | | | | 5.49E-06 | 9.2E-06 | 1.41E-05 | 1.88E-05 | 2.54E-05 | 3.62E-05 | 5.18E-05 |
| A0000007 | | | | | | 7.14E-06 | 1.21E-05 | 1.86E-05 | 2.41E-05 | 3.27E-05 | 4.63E-05 | 6.52E-05 |
| A0000008 | | | | | | 1.03E-05 | 1.64E-05 | 2.4E-05 | 3.26E-05 | 4.39E-05 | 6.13E-05 | 8.42E-05 |
| A0000009 | | | | | | 8.25E-06 | 1.15E-05 | 1.4E-05 | 1.62E-05 | 1.99E-05 | 2.56E-05 | 3.56E-05 |
| MEP001 | A0000002 | A0000006 | | 0 PAL031 | 0.56 / 0.34 / 0.1 | 4.86E-06 | 8.85E-06 | 1.35E-05 | 1.8E-05 | 2.41E-05 | 3.54E-05 | 5.04E-05 |
| MEP002 | A0000005 | A0000003 | | 0 PRF028 | 0.304 / 0.646 / 0.05 | 1.05E-05 | 1.68E-05 | 2.45E-05 | 3.24E-05 | 4.26E-05 | 5.78E-05 | 7.8E-05 |
| MEP003 | A0000005 | A0000008 | | 0 PRF063 | 1.1726 / -0.0926 / -0.08 | 8.68E-06 | 1.44E-05 | 2.21E-05 | 3.1E-05 | 4.19E-05 | 5.78E-05 | 7.97E-05 |
| MEP004 | A0000006 | A0000009 | A0000002 | PAL037 | 0.306 / -0.0530 / 0.647 / 0.1 | 4.89E-06 | 9.38E-06 | 1.46E-05 | 1.93E-05 | 2.55E-05 | 3.67E-05 | 5.17E-05 |
| MEP005 | A0000008 | A0000005 | A0000003 | PAL006 | 0.6062 / 0.314 / 0.1198 / -0.04 | 1.11E-05 | 1.76E-05 | 2.56E-05 | 3.44E-05 | 4.59E-05 | 6.33E-05 | 8.63E-05 |
| MEP006 | A0000002 | A0000006 | A0000005 | PRF025 | 0.273 / 0.4170 / 0.22 / 0.09 | 9.96E-06 | 1.52E-05 | 2.16E-05 | 2.8E-05 | 3.54E-05 | 4.75E-05 | 6.44E-05 |

BANQUE DE DONNEES SPECTRALES ELARGIE EEI

FIGURE 7

EP 3 155 402 B1

*FIG. 8*

EP 3 155 402 B1

| Name | Kcy | Ksatu | Karo | Kiso | Kene | xxxx |
|---|---|---|---|---|---|---|
| CRK0071 | 124.2982 | 30.2213 | 6.1022 | 20.4044 | 22.6714 | |
| CRK0075 | 123.6416 | 30.3821 | 6.0959 | 20.0519 | 22.6293 | |
| CRK0098 | 123.3631 | 29.719 | 6.1168 | 21.0968 | 22.9267 | |
| CRK0102 | 122.6272 | 29.1777 | 6.1166 | 20.8441 | 23.3606 | |
| CRK0116 | 120.3105 | 29.5099 | 6.1134 | 21.0583 | 23.0629 | |
| HVY0068 | 144.4259 | 52.0212 | 5.9475 | 22.2027 | 13.5996 | |
| HVY0088 | 141.8204 | 47.8997 | 6.0091 | 23.0564 | 14.2034 | |
| HVY0093 | 143.0184 | 49.2953 | 5.9834 | 22.8627 | 14.0157 | |
| HVY0100 | 142.1157 | 48.486 | 5.9932 | 22.8486 | 14.183 | |
| HVY0106 | 143.3684 | 50.5179 | 5.9642 | 22.8179 | 13.8181 | |
| KER0072 | 114.2108 | 61.7041 | 5.8061 | 22.7724 | 13.9045 | |
| KER0076 | 112.7754 | 59.6154 | 5.8289 | 23.7607 | 14.191 | |
| KER0079 | 113.9782 | 56.389 | 5.8904 | 22.7878 | 14.299 | |
| KER0080 | 113.2538 | 57.5513 | 5.8681 | 23.0583 | 14.2554 | |
| KER0082 | 114.2399 | 62.3519 | 5.8108 | 23.6506 | 13.8976 | |
| LGT0067 | 130.8834 | 53.1217 | 5.9227 | 22.969 | 13.9002 | |
| LGT0087 | 129.4485 | 49.2411 | 5.9723 | 23.7952 | 14.4472 | |
| LGT0096 | 129.5462 | 48.6752 | 5.9809 | 23.382 | 14.5025 | |
| LGT0099 | 129.1717 | 48.8969 | 5.9775 | 23.6349 | 14.5186 | |
| LGT0105 | 129.4355 | 50.1752 | 5.9592 | 23.4217 | 14.267 | |

Tableau d'Agrégats

FIGURE 9

| Absorbance Nom | %Poids | Germe 1 | Germe 2 | Germe 3 | 4764 | 4760 | 4756 | 4752 | 4748 | 4744 | 4740 | 4736 | 4732 | MON | RON |
|---|---|---|---|---|---|---|---|---|---|---|---|---|---|---|---|
| A0000001 | | | | | 7.78E-06 | 1.29E-05 | 1.88E-05 | 2.42E-05 | 3.16E-05 | 4.42E-05 | 6.18E-05 | 8.01E-05 | 9.88E-05 | 85.1 | 94.2 |
| A0000002 | | | | | 8.08E-06 | 1.37E-05 | 1.96E-05 | 2.5E-05 | 3.18E-05 | 4.46E-05 | 6.09E-05 | 7.91E-05 | 9.8E-05 | 85.3 | 94.7 |
| A0000003 | | | | | 8.78E-06 | 1.52E-05 | 2.23E-05 | 2.97E-05 | 3.99E-05 | 5.53E-05 | 7.54E-05 | 9.68E-05 | 0.000118 | 85 | 94 |
| A0000004 | | | | | 7.46E-06 | 1.26E-05 | 1.81E-05 | 2.47E-05 | 3.29E-05 | 4.71E-05 | 6.55E-05 | 8.47E-05 | 0.000104 | 85 | 93.5 |
| A0000005 | | | | | 1.01E-05 | 1.59E-05 | 2.39E-05 | 3.28E-05 | 4.34E-05 | 5.93E-05 | 8.08E-05 | 0.000104 | 0.000128 | 85.3 | 95.1 |
| A0000006 | | | | | 5.49E-06 | 9.2E-06 | 1.41E-05 | 1.88E-05 | 2.54E-05 | 3.62E-05 | 5.18E-05 | 6.97E-05 | 8.68E-05 | 84.4 | 92.8 |
| A0000007 | | | | | 7.14E-06 | 1.21E-05 | 1.86E-05 | 2.41E-05 | 3.27E-05 | 4.63E-05 | 6.52E-05 | 8.49E-05 | 0.000104 | 85 | 93.5 |
| A0000008 | | | | | 1.03E-05 | 1.64E-05 | 2.4E-05 | 3.26E-05 | 4.39E-05 | 6.13E-05 | 8.42E-05 | 0.000109 | 0.000136 | 85.5 | 95.1 |
| A0000009 | | | | | 8.25E-06 | 1.15E-05 | 1.4E-05 | 1.62E-05 | 1.99E-05 | 2.56E-05 | 3.56E-05 | 4.94E-05 | 6.62E-05 | 85.4 | 95 |
| I2G022 | 0.564 0.436 | A0000003 | A0000006 | | 7.35E-06 | 1.26E-05 | 1.88E-05 | 2.49E-05 | 3.36E-05 | 4.7E-05 | 6.51E-05 | 8.5E-05 | 0.000105 | 84.73846 | 93.47693 |
| I2G011 | 0.654 0.346 | A0000009 | A0000001 | | 8.09E-06 | 1.2E-05 | 1.57E-05 | 1.9E-05 | 2.4E-05 | 3.21E-05 | 4.47E-05 | 6E-05 | 7.75E-05 | 85.29608 | 94.72287 |
| I2G036 | 0.44 0.56 | A0000008 | A0000004 | | 8.69E-06 | 1.43E-05 | 2.07E-05 | 2.81E-05 | 3.77E-05 | 5.33E-05 | 7.38E-05 | 9.54E-05 | 0.000118 | 85.21994 | 94.20381 |
| I3G038 | 0.747 0.258 -0.005 | A0000008 | A0000002 | A0000004 | 9.71E-06 | 1.57E-05 | 2.29E-05 | 3.06E-05 | 4.09E-05 | 5.7E-05 | 7.83E-05 | 0.000101 | 0.000126 | 85.45096 | 95.00502 |
| I3G025 | 0.5825 0.3020 0.1155 | A0000008 | A0000005 | A0000003 | 1E-05 | 1.61E-05 | 2.38E-05 | 3.23E-05 | 4.33E-05 | 6E-05 | 8.22E-05 | 0.000106 | 0.000131 | 85.38181 | 94.97284 |
| I3G019 | 0.094 -0.00047 0.00647 | A0000004 | A0000005 | A0000007 | 7.43E-06 | 1.25E-05 | 1.81E-05 | 2.46E-05 | 3.28E-05 | 4.7E-05 | 6.54E-05 | 8.46E-05 | 0.000104 | 84.99859 | 93.49245 |

BANQUE DE DONNEES SPECTRALES ELARGIE E – avec caractérisation

FIGURE 10

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0742900 A

**Littérature non-brevet citée dans la description**

- **ESPINOSA A et al.** USE NIR TECHNOLOGY TO OPTIMIZE PLANT OPERATIONS. *HYDROCARBON PROCESSING,* 01 Février 1995, 86-92